# EUROPÄISCHE PATENTSCHRIFT

(11) **EP 2 477 993 B1**
(45) Veröffentlichungstag und Bekanntmachung des Hinweises auf die Patenterteilung: **24.06.2015**
(21) Anmeldenummer: 10747788.7
(22) Anmeldetag: 12.08.2010
(51) Int. Cl.: C07D 498/04, A61K 31/424, A61P 31/04

(54) **SUBSTITUIERTE CLAVULANSÄURE**
SUBSTITUTED CLAVULANIC ACID
ACIDE CLAVULANIQUE SUBSTITUÉ

(30) Priorität: 15.09.2009 AT 14582009
(43) Veröffentlichungstag der Anmeldung: 25.07.2012
(73) Patentinhaber: Nabriva Therapeutics AG, 1112 Wien (AT)
(72) Erfinder: BULUSU, Atchyuta, Rama, Chandra, Murty, A-2380 Perchtoldsdorf (AT)
(74) Vertreter: Schwarz, Albin
(86) Internationale Anmeldenummer: PCT/AT2010/000297
(87) Internationale Veröffentlichungsnummer: WO 2011/032192

(56) Entgegenhaltungen:
- READING C ET AL: "STRUCTURE-ACTIVITY RELATIONSHIPS AMONGST BETA-LACTAMASE INHIBITORS" JOURNAL OF ENZYME INHIBITION, NEW YORK, NY, US, Bd. 1, Nr. 2, 1. Januar 1986 (1986-01-01), Seiten 83-104, XP000985066

## Beschreibung

Die vorliegende Erfindung bezieht sich auf organische Verbindungen und deren Zwischenverbindungen, und deren Anwendungen sowie eine pharmazeutische Zusammensetzung umfassend eine solche Verbindung oder deren Zwischenverbindung.

Da Bakterien gegenüber Antibiotika resistent werden, besteht im Stand der Technik ein steigender Bedarf an der Entwicklung wirksamerer Antibiotika. Es wurde festgestellt, dass Clavulansäure ein sehr starker β-Lactamase-Hemmer ist. Insbesondere eine Kombination von Clavulansäure und Amoxicillin erwies sich als äußerst wirksam und wird als Augmentin vermarktet.

In Reading C et al, Journal of Enzyme Inhibition, New York NY, US, Vol. 1, Nr. 2, 1. Januar 1986, Seiten 83-104 sind Struktur-Aktivitätsbeziehungen in Beta Lactamase Inhibitoren betreffend u.a. Clavulansäurederivate, beschrieben.

Das der vorliegenden Erfindung zugrunde liegende technische Problem besteht in der Bereitstellung von Verbindungen, namentlich Clavamen, die allein oder in Kombination mit anderen Antibiotika eine verbesserte antibiotische Wirksamkeit haben und die Nachteile des Standes der Technik überwinden.

Dieses Problem wurde durch eine Verbindung der Formel I 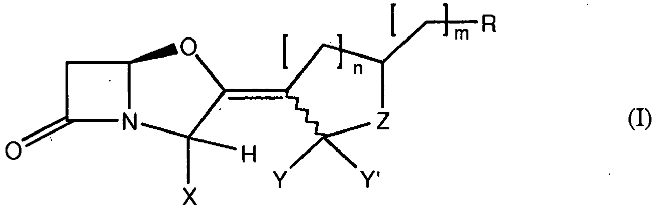 wobei
- X: COOH oder ein Salz davon,
COOR₁, wobei R₁ optional substitutiertes C₁₋₄ Alkyl darstellt,
CONR₂R₃, wobei R₂ und R₃ unabhängig voneinander H oder C₁₋₅ Alkyl, das optional ein oder mehrere Heteroatome ausgewählt aus O, S und N enthält und/oder optional substituiert ist, oder
CON(R₂)-CH(R₄)-R₅, wobei
R₂ wie oben definiert ist,
R₄ eine Gruppe ist, die gewönlich in der α Stellung von α-Aminosäuren vorkommt,
und R₅
COOH oder ein Salz davon; COOR₁, wobei R₁ wie oben definiert ist; oder CONR₂R₃, wobei R₂ and R₃ wie oben definiert sind,
darstellt,
- Y and Y': beide H sind oder zusammen =O darstellen,
- Z O, NR₂: oder N-CH(R₄)-R₅ ist, wobei R₂, R₄ und R₅ wie oben definiert sind,
- n: 1 oder 2 ist
- m: eine Zahl von 0 bis 4 ist,
- R: H, oder
eine gesättigte oder ungesättigte Kette mit 1 bis 8 Kohlenstoffatomen, die optional durch einen 5 oder 6-gliedrigen, nichtaromatischen Ring, der ein oder mehrere Heteroatome, ausgewählt aus O, S und N, enthält, oder durch Aryl, Alkyl oder Cycloalkyl substituiert ist, optional eine oder mehrere Heteroatome ausgewählt aus O, S und N enthält, und/oder ein- oder mehrfach substituiert ist durch ein oder mehrere Heteroatome ausgewählt aus O, S und N; Halogene Cl, Br, I und F und/oder Azido N₃,
ist,
gelöst.

In der Formel I sowie in den Formeln unten verkörpert eine an einer Doppelbindung angebrachte gezackte Bindung ( 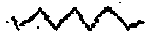 oder 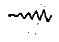 -Bindung) sowohl das E- als auch das Z-Isomer der spezifischen Verbindung. Daher werden sowohl das E- als auch das Z-Isomer durch diese Formel beschrieben.

Bei einer bevorzugten Ausführungsform ist das Salz ein pharmazeutisch akzeptables Salz, vorzugsweise ein Kalium Salz.

Bei einer weiteren bevorzugten Ausführungsform stellt R₁ eine Methyl, das vorzugsweise durch eine Pivaloyloxygruppe substituiert ist, dar.

Vorzugsweise sind R₂ und R₃ unabhängig voneinander durch C₁₋₄ Alkyl oder C₅₋₆ Cycloalkyl substituiert.

Bei einer bevorzugten Ausführungsform stellt R₄ Methyl- oder eine Benzylgruppe dar.

Bei einer weiteren bevorzugten Ausführungsform stellt R eine gesättigte oder ungesättigte Kette mit 1 bis 8 Kohlenstoffatomen, die durch einen 5 oder 6-gliedrigen nichtaromatischen Ring, der eine oder mehrere Heteroatome ausgewählt aus O, S und N enthält, Aryl, C₁₋₄ Alkyl oder C₅₋₆ Cycloalkyl substituiert ist, dar.

Bei einer weiteren bevorzugten Ausführungsfom stellt R eine gesättigte oder ungesättigte Kette mit 1 bis 8 Kohlenstoffatomen dar, die durch ein oder mehrere Heteroatome ausgewählt aus O, S und N substituiert ist, wobei die O und S Heteroatome als Ester, Carbonate oder Äther, und die N Heteroatome alkyliert oder als Amide oder Carbamate geschützt sind.

Eine besonders bevorzugte Verbindung der Formel I ist in Formel III definiert.

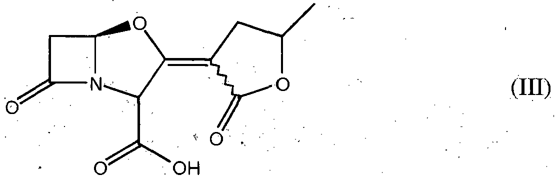

Im folgenden werden Zwischenverbindungen der Formel II 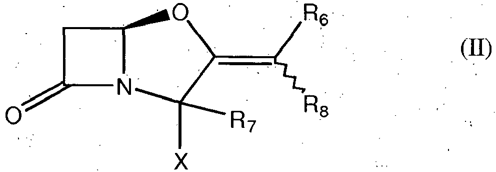 beschrieben, wobei
X wie für Formel I definiert ist,
entweder R₆ oder R₇ H ist, mit der Maßgabe, dass
wenn R₇ H ist, R₆ eine gesättigte oder ungesättigte Kette mit 1 bis 11 Kohlenstoffatomen ist, die optional durch einen 5 oder 6-gliedrigen nichtaromatischen Ring, der ein oder mehrere Heteroatome aus O, S und N enthält, oder durch Aryl, Alkyl oder Cycloalkyl substituiert ist, die optional ein oder mehrere Atome ausgewählt aus O, S und N enthält, und/oder durch ein oder mehrere Atome ausgewählt aus O, S und N, Halogene Cl, Br, I und F und/oder Azido N₃ substituiert ist,
oder vice versa,
und
R₈ ist COOH, CH₂OH oder CHO ist.

Zwischenverbindungen der Formel II sind als Ausgangsmaterialien für die Erstellung der Verbindungen von Formel I verwendbar. Allerdings zeigen diese Verbindungen auch antimikrobielle Wirksamkeit und sind allein oder in Kombination mit anderen Antibiotika als Antibiotika verwendbar.

Bei einer bevorzugten Ausführungsform stellt R₆, beziehungsweise R₇ eine gesättigte oder ungesättigte Kette mit 1 bis 11 Kohlenstoffatomen, die durch einen 5 oder 6-gliedrigen nichtaromatischen Ring, der ein oder mehrere Heteroatome ausgewählt aus O, S und N enthält, oder durch Aryl, C₁₋₄ Alkyl oder C₅₋₆ Cycloalkyl substituiert ist, dar.

Bei einer weiteren bevorzugten Ausführungsform stellt R₆ beziehungsweise R₇ eine gesättigte oder ungesättigte Kette mit 1 bis 11 Kohlenstoffatomen, die durch ein oder mehrere Heteroatome ausgewählt aus 0, S und N, substitiuert ist, wobei die O und S Herteroatome als Ester, Carbonate oder Äther, und die N Heteroatome alkyliert oder als. Amide oder Carbamate geschützt sind, dar.

Eine besonders bevorzugte Verbindung der Formel II ist durch die Formel IV definiert. 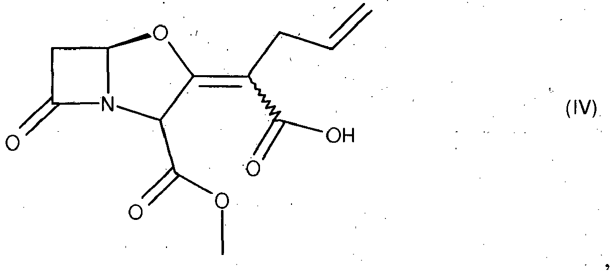 eine weitere bevorzugte

Verbindung der Formel II ist durch die Formel V 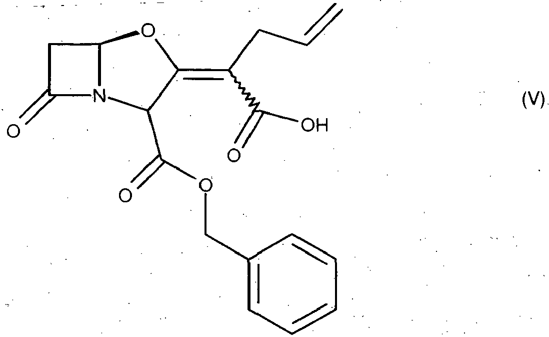 definiert. Verbindungen der Formeln (IV) und (V) sind neu und ebenfalls Gegenstand der vorliegenden Erfindung.

Die Verbindungen der vorliegender Erfindung können in Form als Isomere und deren Gemische z.B. inklusive Diastereoisomeren und deren Gemische existieren. Isomerengemische können nach Bedarf getrennt werden, z.B. mit einer konventionellen Methode, um reine Isomeren zu erhalten. Die vorliegende Erfindung beinhaltet eine Verbindung der vorliegenden Erfindung in jeder isomeren Form und in jedem isomeren Gemisch.

Die Verbindungen der vorliegenden Erfindung können auch in Form von Solvaten, z.B. Hydraten existieren.

In einem weiteren Aspekt stellt die Erfindung Verbindungen der Formel I und der Formel IV und V zur Verwendung als Medikament zur Verfügung.

Die Verbindungen der vorliegenden Erfindung haben sich als β-Lactamase Inhibitoren, antimikrobielle Substanzen und Antibiotika verwendbar erwiesen, allein oder in Kombination mit anderen Antibiotika.

Die Verbindungen der vorliegenden Erfindung sind im Speziellen dadurch nützlich, dass bei der Verabreichung in Säugetieren die Effektivität eines coadministiierten β-Lactam-Antibiotikums gegen β-lactamaseproduzierende Bakterien gesteigert wird.

Die Verbindungen der vorliegenden Erfindung können allein oder in Kombination(therapie) mit ß-Lactam Antibiotika angewendet werden, um Infektionskrankheiten zu behandeln, unter anderem Erkrankungen der Atemwege, Harnwege und Weichteile in Menschen.

In einem weiteren Aspekt stellt die Erfindung eine pharmazeutische Zusammensetzung, welche eine Verbindung der Formel I und/oder der Formeln IV oder V umfasst und die optional zusätzlich noch ein ß-Lactamantibiotikum von der Klasse der Penicilline oder Cephalosporine umfasst. Typischerweise ist bei einer Kombination in der pharmazeutischen Zusammensetzung das Gewichtsverhältnis des β-Lactamantibiotikums zu der Verbindung der vorliegeneden Erfindung von ca. 15:1 bis ca. 1:1.

Pharmazeutische Zusammensetzungen der vorliegenden Erfindung können für die Behandlung von Infektionskrankheiten in Säugetieren angewendet werden. Bakterielle Infektionskrankheiten, die mit den Verbindungen und pharmazeutischen nicht darauf eingeschränkt, Atemwegserkrankungen inklusive nicht Krankenhaus assoziierter Lungenentzündungen, akute Exazerbation von chronischer Bronchitis und akute bakterielle Sinusitis, verursacht durch Atemwegskeime wie z.B. *Haemophilus influenzae* und *Moraxella calarrhalis* inklusive antibiotisch resistenter Isolate.

Weitere bakterielle Infektionskrankheiten, die mit der pharmazeutischen Kompositionen der vorliegender Erfindung zusammen mit einem Antibiotikum behandelbar sind, inkludieren, beschränken sich jedoch nicht auf, pediatrische Otitis Media, Sinusitis, Pneumoniae, akute Exazerbation von Bronchitis in Erwachsenen verursacht durch *H. influenzae* oder *Streptococcus pneumoniae,* inklusive medikanientenresistente *S*. *pneumonie* z.B. penicillinresistente *S*. *pneumoniae,* Weichteilinfektionen verursacht durch *E. coli, Klebsiella pneumoniae, Enterobacter spp.* und alle anderen Mitglieder der Familie *Enterobacteriaceae,* und Infektionen verursacht durch β-Lactamase produzierende, Methicillin susceptible Staphylococci und β-Lactamase produzierende Anaeroben.

Die passende Dosis für die Behandlung variiert natürlich z.B. abhängig von den chemischen Eigenschaften und den pharmakokinetischen Daten der eingesetzten Verbindung der vorliegenden Erfindung, und dem individuell Behandelten, der Art der Anwendung und der Natur und Heftigkeit des zu behandelnden Leidens. Indessen liegt die tägliche Dosis für eine zufriedenstellende Wirkung in grösseren Säugetieren, z.B. Menschen, im allgemeinen im Bereich von ca. 0.01 g bis zu ca. 2.0 g einer Verbindung der vorliegenden Erfindung, wobei die Dosis vorteilhaft, z.B. in geteilten Protionen, bis zu vier mal pro Tag verabreicht werden kann.

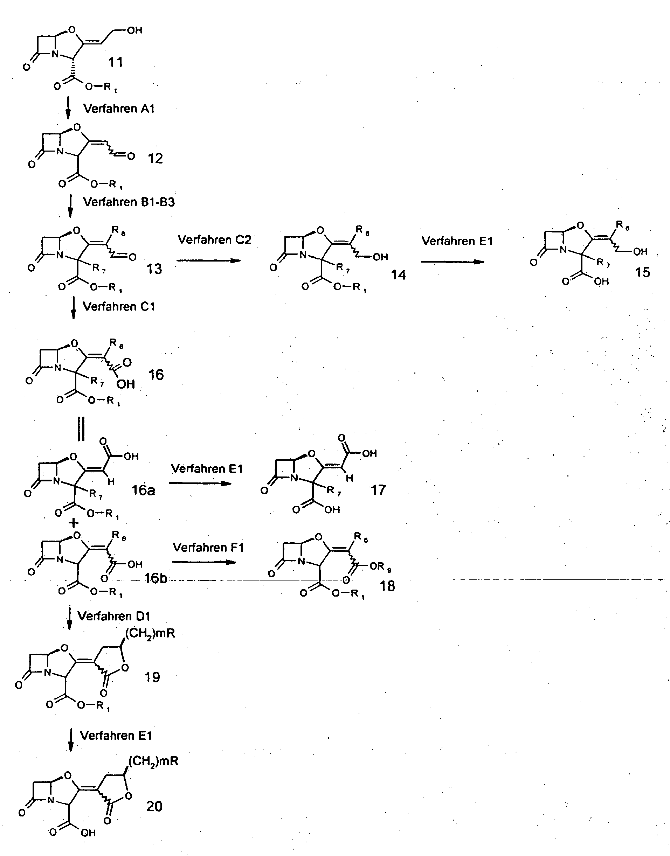

Das Verfahren zur Herstellung der obenstehend identifizierten Verbindungen wird durch das Reaktionsschema Fig.1 veranschaulicht. Es versteht sich, dass es sich bei dem Reaktionsschema nur um eine bevorzugte Ausführungsform des Verfahrens zur Bereitstellung von Verbindungen gemäß der vorliegenden Erfindung handelt.

Die Verbindung 11, z. B., kann durch Veresterung von Clavulansäure erhalten werden. Anschließend wird die Verbindung 11 gemäß dem Verfahren A1 mit einem geeigneten Oxidationsmittel oxidiert. Eine Möglichkeit zur Umwandlung der Hydroxylgruppe der Verbindung 11 in eine Aldehydgruppe ist das Dess-Marlin-Oxidationsverfahren, wobei das Dess-Martin-Periodinan-Reagens eingesetzt wird.

Gemäß dem Verfahren B1 bis B3 wird die Verbindung 12 mit einem geeigneten nukleophilen Reagens umgesetzt, um die Verbindung 13 zu bilden. Beim Verfahren B1 handelt es sich vorzugsweise um eine Palladium-katalysierte nukleophile Substitutionsreaktion.

Beim Verfahren B2 reagieren 1,4-Dibrombuten und K₂CO₃ vorzugsweise mit der Verbindung 12, wodurch die substituierte Verbindung 13 erhalten wird. Anschließend kann das im Verfahren B2 erhaltene Produkt im Verfahren B3 in das entsprechende Azidoderivat umgewandelt werden, wobei vorzugsweise Tetrabutylarnmoniumazid als Reagens verwendet : wird.

Um den Alkohol 14 zu erhalten, kann die Verbindung 13 mit einem geeigneten Reduktionsmittel reduziert werden. Das Verfahren C2 kann unter Verwendung von NaBH₄ als Reduktionsmittel durchgeführt werden. Gegebenenfalls kann die Estergruppe des Alkohols 14 im Verfahren E1 gespalten werden, um die entsprechende Carbonsäure 15 zu bilden.

Gemäß dem Verfahren C1 wird Aldehyd 13 mit einem geeigneten Oxidationsmittel oxidiert, wodurch das Cärbonsäurederivat 16 gebildet wird. Bei einer bevorzugten Ausführungsform können die Carbonsäurederivate 16a und 16b in der obenstehend erwähnten Oxidationsreaktion als Mischung beider E- und Z-Isomere gebildet werden. Falls der Substituent R₆ Wasserstoff, wie in Verbindung 16a ist, entspricht die Konfiguration der Doppelbindung der Alkenylkomponente vorzugsweise Z. Falls der Substituent R₇ Wasserstoff, wie in Verbindung 16b ist, ist das erhaltene Produkt vorzugsweise nur ein Isomer von den möglichen E oder Z-Isomeren.

Beim Verfahren E1 wird die Estergruppe des Z-Isomers 16a mit einem geeigneten Reagens gespalten, wodurch das Dicarbonsäurederivat 17 gebildet wird. Die Verbindung 16b kann gegebenenfalls durch ein geeignetes Veresterungsverfahren in den Diester-18 umgewandelt werden (Verfahren F1, R₉ ist definiert wie für R₁).

Beim Verfahren D1 wird der 5-gliedrige Ring der Verbindung 19 durch Umsetzung der Verbindung 16b mit einem geeigneten Ringbildungsmittel gebildet. In der Ringbildungsreaktion D1 ist der Substituent R₆ der Verbindung 16b vorzugsweise eine Allylgruppe oder eine substituierte Allylgruppe. Die Cyclisierungsreaktion wird bevorzugt mit Iod katalysiert, wodurch die Verbindung 19 gebildet wird, wobei R eine iodierte Alkylgruppe oder eine substituierte Alkylgruppe ist.

Gemäß einem Verfahren kann die Verbindung 19 enthalogeniert werden, wobei bevorzugt eine Palladium-katalysierte Dehalogenierungsreaktion zur Anwendung kommt.

Alternativ kann diese Verbindung, wenn R der Verbindung 19 eine kodierte Alkylgruppe oder eine substituierte Alkylgruppe ist, gemäß einem anderen Verfahren in das entsprechende Azidoderivat umgewandelt werden.

Die Verbindung 20 kann durch Spaltung der Estergruppe der Verbindung 19 erhalten werden. Diese Spaltungsreaktion ist vorzugsweise eine reduzierende Esterspaltungsreaktion. Falls R eine Alkylgruppe ist, die durch eine Azidogruppe substituiert ist, wird diese Azidogruppe vorzugsweise gleichzeitig ebenfalls reduziert.

Der Fachmann erkennt mehrere Alternativen bei den beschriebenen spezifischen Reaktionen, mit denen dieselben Reaktionsprodukte erhalten werden können.

Die nachfolgenden Beispiele veranschaulichen die Erfindung. Sie sind je doch nicht einschränkend.

### Beispiele:

Alle Temperaturen sind in Grad Celsius (°C) angegeben und nicht korrigiert. In den magnetischen Kernresonanzspektren sind alle chemischen Verschiebungswerte in ppm angegeben.

Die folgenden Abkürzungen werden verwendet: s: Singulett; d; Dublett; t; Triplett; q: Quartett; m: Multiplett; dd: Dublett eines Dubletts; dt: Dublett eines Tripletts; dq: Dublett eines Quartetts; ddt: Dublett eines Dublett eines Tripletts; br: breit; Abq: AB-Quartett; DMSO: Dimethylsulfoxid; aq: wässrig; rt: Raumtemperatur.

### Herstellung von Zwischenverbindungen (Ausmangsmaterialien) für Verbindungen der Beispiele 1-22

### Beispiel A

### Mischung von (5R)-7-Oxo-3-(2-oxo-ethyliden)-4-oxa-1-aza-bicyclo[3.2.0]heptan-2-carbonsäurebenzylester und (R)-3-(2-Hydroxyvinyl)-7-oxo-4-oxa-1-aza-bicyclo[3.2.0]hept-2-en-2-carbonsäurebenzylester

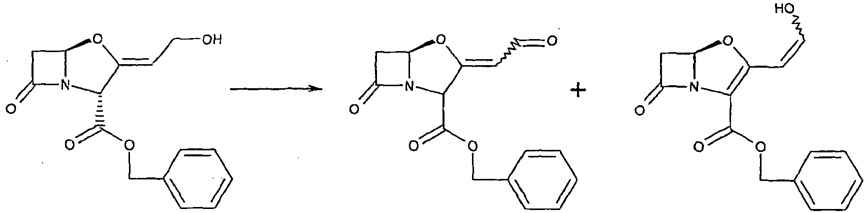

Eine Mischung aus (2R,5R)-3-[2-Hydroxy-eth-(Z)-yliden]-7-oxo-4-oxa-1-aza-bicyclo[3.2.0]heptan-2-carbonsäurebenzylester (58,86 g), 1200 ml trockenem Dichlormethan, 59 g 4 A°-Molekularsieben und Dess-Martin-Periodinan-(1,1,1-Tris(acetyloy)-1,1-diydro-1,2-benziodoxol-3(1H)-on) (76,52 g) wird bei 0-5°C 1,5 Stunden lang und danach bei 10°C 40 Minuten lang gerührt. Die Mischung wird durch einen Sintertrichter gefiltert, und das Filtrat wird unter mechanischem Rühren in einen 10-Liter-Reaktor gegossen, der eine Lösung von 503,2 g Natriumthiosulfat.5H₂O, gelöst in 2370 ml gesättigtem wässrigem NaHCO₃, enthält. Anschließend werden 600 ml Diethylether hinzugefügt, und die Mischung wird 20 Minuten lang gerührt. 1500 ml Ethylacetat und 500 ml Diethylether werden hinzugefügt und für weitere 2 Minuten gerührt. Die wässrige Phase wird entfernt, und die organische Phase wird mit 600 ml wässrigem gesättigtem NaHCO₃ und mit 600 ml Kochsalzlösung jeweils zweimal gewaschen, getrocknet (MgSO₄), filtriert und bei Raumtemperatur und unter Vakuum vom Lösungsmittel abgezogen, um ein Öl zu ergeben. Das Öl wird unter Verwendung von Toluol / Ethylacetat (6/1), enthaltend 0,05%ige Essigsäure, an einer SiO₂-Säule chromatographiert. Die Fraktionen, welche die gewünschte Verbindung enthalten, werden kombiniert und mit 400 ml wässrigem 5%igem NaHCO₃ und jeweils zweimal mit 400 ml Kochsalzlösung gewaschen, getrocknet und von den Lösungsmitteln abgezogen. Die Titelverbindung wird als Öl mit den untenstehenden Kenndaten erhalten.

**PatAZ9433h ¹H-NMR** (DMSO-d₆, 400,MHz):δ 11,07 (br. s); 9,84 (d, J = 8,2 Hz); 9,59 (d, J = 6,8 Hz); 7,41-7,32 (m); 6,33 (d, J = 1,1 Hz); 6,10 (d, J = 12,4 Hz); 6,07 (d, J = 2,7 Hz); 5,99 (d, J = 2,7 Hz); 5,96 (d, J = 2,4 Hz); 5,88 (s); 5,83 (dd, J = 6,9, 1,2 Hz); 5,35 (dd, J = 8,2, 0,6 Hz); 5,23-5,18 (überlappende Multipletten); 3,84-3,75 (überlappende Mliltipletten); 3,49 (d, J = 17,2 Hz); 3,41 (d, J = 17,2 Hz); 3,35 (d, J = 17,2 Hz).

### Beispiel B

### Mischung von (5R)-7-Oxo-3-(2-oxo-ethyliden)-4-oxa-1-aza-bicyclo[3.2.0]heptan-2-carbonsäüremethylester und (R)-3-(2-Hydroxyvinyl)-7-oxo-4-oxa-1-aza-bicyclo[3.2.0]hept-2-en-2-carbonsäuremethylester

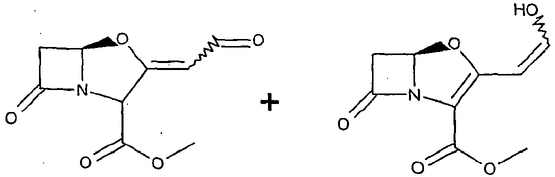

Analog zu dem im Beispiel A beschriebenen Verfahren, jedoch unter Verwendung von (2R,5R)-3-[2-Hydroxy-eth-(Z)-yliden]-7-oxo-4-oxa-1-aza-bicyclo[3.2.0]heptan-2-carbon-säuremethylester, wird die Titelverbindung als Öl mit den untenstehenden Kenndaten erhalten.

**PatAZ8242I ¹H-NMR** (DMSO-d₆, 400 MHz): δ 11,01 (br. s); 9,85 (d, J = 8,2 Hz); 9,58 (d, J = 6,8 Hz); 7,34 (d, J = 12,3 Hz); 6,24 (d, J = 1,3 Hz); 6,10 (d, J = 12,6 Hz); 6,07 (d, J = 2,4 Hz); 5,99 (d, J = 2,4 Hz); 5,95 (d, J = 2,0 Hz); 5,83 (dd, J = 1,3, 6,9 Hz); 5,80 (d, J = 0,8 Hz); 5,36 (dd, J = 0,9, 8,2 Hz); 3,81 & 3,77 (zwei überlappende Dubletten von Dubletten); 3,74 (s); 3,73 (s); 3,67 (s); 3,47 (d, J = 17,2 Hz); 3,40 (d, J = 17,2 Hz); 3,35 (d, J = 17,2 Hz).

### Beispiel C

### Mischung von (S5)-7-Oxo-3-(2-oxo-ethyliden)-4-oxa-1-aza-bicyclo[3.2.0]heptan-2-carbonsäure-2,2-dimethylpropionyloxymethylester und (R)-3-(2-Hydroxyvinyl)-7-oxo-4-oxa-1-aza-bicyclo[3.2.0]hept-2-en-2-carbonsäure-2,2-dimethylpropionyloxymethylester

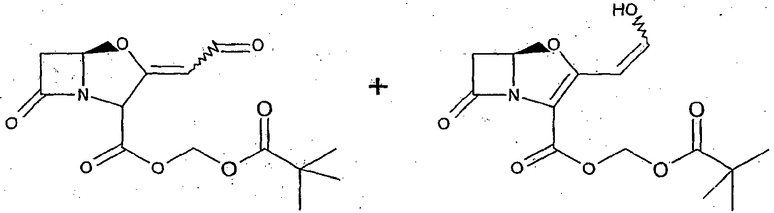

Analog zu dem im Beispiel A beschriebenen Verfähren, jedoch unter Verwendung von (2R,5R)-3-[2-Hydroxy-eth-(Z)-yliden]-7-oxo-4-oxa-1-aza-bicyclo[3.2.0]heptan-2-carbonsäure-2,2-dimethylpropionyloxymethylester, wird die Titelverbindung als Öl mit den untenstehenden Kenndaten erhalten.

**PatAZ9481H ¹H-NMR** (DMSO-d₆, 400 MHz): δ 11,25 (br. s); 9,84 (d, J = 7,9 Hz); 9,55 (d, J = 6,6 Hz); 7,42 (d, J = 12,1 Hz); 6,32 (d, J = 1,3 Hz); 6,06 (d, J = 12,4 Hz); 5,98-5,96 (überlappende Multipletten); 5,91 (d, J = 0,9 Hz); 3,84-3,76 (überlappende Multipletten); 3,49 (d, J = 17,2 Hz); 3,42 (d, J = 17,2 Hz); 3,36 (d, J = 17,2 Hz).

### Beispiel D

### Benzyl-(4-brombut-2-enyl)-carbaminsäurebenzylester

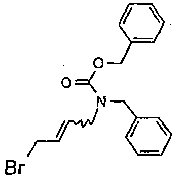

Eine Mischung aus N-Benzyloxycarbonylbenzylamin (50 g), 1,4-Dibrom-2-buten (44 g) und NaH (10,7 g, 60% in Öl) in 350 ml DMF wird bei 0°C eine Stunde lang und danach bei 25°C 3 Stunden lang gerührt. Die Reaktionsmischung wird einer wässrigen Aufarbeitung unterzogen, und der erhaltene Rückstand wird durch Chromatographie an SiO₂ gereinigt. Die Titelverbindung wird als Öl mit den untenstehenden Kenndaten erhalten.

**Pat AZ 1840K ¹H-NMR** (400 MHz, DMSO): 7,42-7,16 (m, 10 H); 5,79 (br s, 2 H); 5,12 (s, 2 H); 4,41 (s, 2 H); 4,10 (br s); 3,85 (br s).

### Beispiele 1, 2 und 3

Beispiel 1: (5R)-3-(1-Formyl-but-3-enyliden)-7-oxo-4-oxa-1-aza-bicyclo[3.2.0]heptän-2-carbonsäurebenzylester
Beispiel 2: (5R)-2-Allyl-7-oxo-3-[2-oxo-ethyliden]-4-oxa-1-aza-bicyclo[3.2.0]heptan-2-carbonsäurebenzylester
Beispiel 3: (5R)-2-Allyl-3-[1-formyl-but-3-enyliden]-7-oxo-4-oxa-1-aza-bicyclo[3.2.0]heptan-2-carbonsäurebenzylester

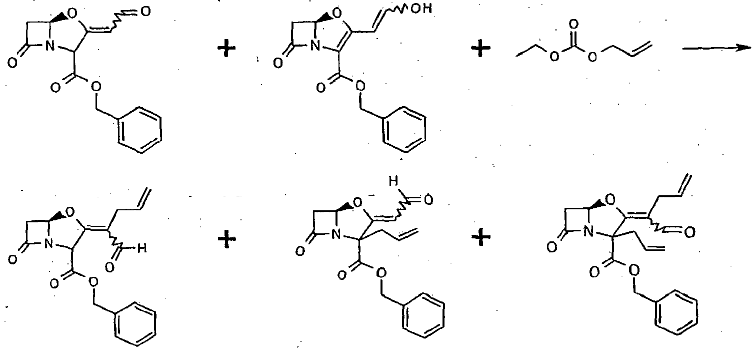

Eine Mischung aus der Verbindung des Beispiels A (32,46 g), 500 ml Dichlormethan, Carbonsäureallylesterethylester (11,13 ml) und Pd(Ph₃P)₄ (3,92 g) wird unter einer Argonatmosphäre für 1 h 15 min gerührt. Das Lösungsmittel wird unter Vakuum entfernt, um einen Rückstand zu erhalten. Der Rückstand wird unter Verwendung von Toluol / t-Butylmethylether (25/1, 20/1 und 15/1) an einer SiO₂-Säule chromatographiert. Die Verbindung des Beispiels 1 wird als Öl mit den untenstehenden Kenndaten erhalten.

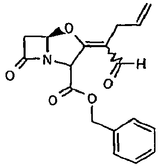

**PatAZ2506i** ¹**H**-**NMR** (DMSO-d₆, 400 MHz): δ 9,62 (s, 1 H); 7,40-7,32 (m, 5 H); 6,47 (s, 1 H); 6,00 (dd, J = 3,1, 0,7 Hz; 1 H); 5,77-5,68 (m, 1 H); 5,20 und 5,23 (zwei Dubletten als ABq, J = 12,6 Hz, 2 H); 4,93-4,87 (m, 2 H); 3,78 (dd, J = 3,1, 17,2 Hz, 1 H); 3,30 (dd, J = 17,2, 0,7 Hz, 1 H); 2,95 (dt, Jd= 5,95 Hz, Jt = 1,4 Hz, 2 H).

Die Verbindung des Beispiels 2 wird als Öl mit den untenstehende Kenndaten erhalten.

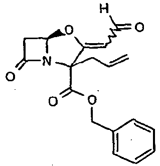

**PatAZ 02505i ¹H-NMR** (DMSO-d₆, 400 MHz): Mischung von Isomeren;
Daten des Hauptisomers: δ 9,84 (d, J = 7,8 Hz, 1 H); 7,40-7,30 (m, 5 H); 5,94 (d, J = 2,8 Hz, 1 H); 5,76-5,67 (m, 1 H); 5,45 (d, J = 8,0 Hz, 1 H); 5,22-5,16 (überlappende Multipletten); 3,70 (dd, J = 2,9, 17,0 Hz; 1 H); 3,41 (d, J = 17,0 Hz, 1 H); 3,10 (dd, J =8,5, 14,6 Hz, 1H); Daten des Nebenisomers: δ 9,86 (d, J = 8,0 dz, 1 H); 7,40-7,30 (m, 5 H); 5,92 (d, J = 2,6 Hz, 1 H); 5,90-5,85 (m, 1 H); 5,36 (d, J = 7,9 Hz, 1 H); 5,26 (d, J = 12,4 Hz, 1 H); 5,22-5,16 (überlappende Multipletten); 5,10 (d, J = 12,5 Hz, 1 H); 3,64 (dd, J = 2,9, 17,0 Hz); 3,40 (d, J = 17,1 Hz); 2,70 (dd, J = 7,1, 14,2 Hz).

Die Verbindung des Beispiels 3 wird als Schaum mit den untenstehende Kenndaten erhalten.

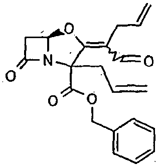

**PatAZ02523i ¹H-NMR** (DMSO-d₆, 500 MHz): δ 9,69 (s, 1 H); 7,38-7,31 (m, 5 H); 5,81 (d, J = 2,3 Hz, 1 H); 5,73-5,63 (m, 2 H); 5,21 & 5,24 (zwei Dubletten als ABq, J = 12,4 Hz, 2 H); 5,18 (dd, J = 1,8, 16,9 Hz, 1 H); 5,13 (dd, J = 1,8, 10,1 Hz, 1 H); 4,88 (dq, Jd = 6,0, Jq = 1,6 Hz, 1 H); 4,86 (t, J = 1,6 Hz, 1 H); 3,62 (dd, J = 2,8, 17,0 Hz, 1 H); 3,53 (ddt, Jd = 6,4, 15,1, Jt = 1,4 Hz, 1 H); 3,42 (dd, J = 0,9, 16,9 Hz, 1 H); 3,18 (dd, J = 7,3, 15,6 Hz, 1 H); 2,98 (ddt, Jd = 6,0, 15,1, Jt = 1,4 Hz, 1 H); 2,93 (ddt, Jd = 6,0, 15, 1, Jt = 1,4 Hz, 1 H).

### Beispiele 4, 5, 6

Beispiel 4: (5R)-3-[1-Formyl-4-phenyl-but-3-enyliden]-7-oxo-4-oxa-1-aza-bicyclo[3.2.0]heptan-2-carbonsäurebenzylester
Beispiel 5: (5R)-7-Oxo-3-[2-oxo-ethyliden]-2-(3-phenylallyl)-4-oxa-1-aza-bicyclo[3.2.0]heptan-2-carbonsäurebenzylester
Beispiel 6 (Isomer der Verbindung des Beispiels 5): (5R)-7-Oxo-3-[2-oxo-ethyliden]-2-(3-phenylallyl)-4-oxa-1-aza-bicyclo[3.2.0]heptan-2-carbonsäurebenzylester

Analog zu dem in den Beispielen 1-3 beschriebenen Verfahren, jedoch unter Verwendung der Verbindung des Beispiels A und von Carbonsäureethylester (E)-3-phenylallylester, wird die Verbindung des Beispiels 4 als Öl mit den untenstehenden Kenndaten

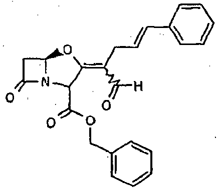

**PatAZ2588i** ¹**H**-**NMR** (DMSO-d₆, 400 MHz): δ 9,66 (s, 1 H); 7,40-7,20 (m, 10 H); 6,50 (s, 1 H); 6,32 (d, J = 15,8 Hz, 1 H); 6,17 (dt, Jd = 15,8, Jt = 6,3 Hz; 1 H); 6,04 (d, J = 2,5 Hz, 1 H); 5,24 & 5,20 (zwei Dubletten als ABq, J = 12.4 Hz, 2 H); 3,78 (dd, J = 3,1, 17,2 Hz, 1 H); 3,31 (d, J = 17,1 Hz, 1 H); 3,12 (d, J = 6,2 Hz, 2 H);
genauso wie die Verbindung des Beispiels 5 als Öl mit den untenstehenden Kenndaten

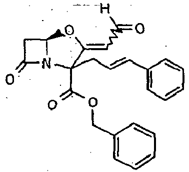

**PatAZ2590i ¹H-NMR** (DMSO-d₆, 400 MHz): δ 9,87 (d, J = 7,8 Hz, 1 H); 7,35-7,20 (m, 10 H); 6,56 (d, J = 15,9 Hz, 1 H); 6,29 (ddd, J = 15,9, 8,4, 5,6 Hz, 1 H); 5,97 (d, J = 2,3 Hz, 1 H); 5,52 (d, J = 7,9 Hz, 1 H); 5,18 (s, 2 H); 3,72 (dd, J = 3,1, 17,0 Hz, 2 H); 3,44 (d, J = 17,1 Hz, 1 H); 3,31 (dd, J = 14,9, 8,6 dz, 1 H); 3,03 (ddd, J = 14,5, 5,6, 1,4 Hz, 1 H);
und die Verbindung des Beispiels 6 als Öl mit den untenstehenden Kenndaten

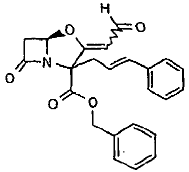

**PatAZ2589i ¹H-NMR (DMSO-d₆, 400 MHz):** δ 9,86 (d, J = 7,9 Hz, 1H); 7,40-7,20 (m, 10 H); 6,55 (d, J = 15,9 Hz, 1H); 6,15 (dt, Jd = 15,6, Jt = 7,5, 1 H); 5,92 (d, J = 2,0 Hz, 1 H); 5,40 (d, J = 7,9 Hz, 1 H); 5,29 (d, J = 12,6 Hz, 1 H); 5,15 (d, J = 12,4 Hz, 1 H); 3,64 (dd, J= 3,0, 17,1 Hz, 1H); 3,40 (dd, J = 0,7, 17,2 Hz, 1 H); 3,02 (ddd, J = 1,1, 7,3, 16,4 Hz, 1 H); (2,88, ddd, J = 1,1, 7,3, 16,4 Hz, 1 H)
erhalten.

### Beispiele 7, 8 und 9

Beispiel 7: (5R)-3-[1-Formyl-5-hydroxy-pent-3-enyliden]-7-oxo-4-oxa-1-aza-bicyclo[3.2.0]heptan-2-carbonsäurebenzylester
Beispiel 8: (5R)-2-(4-Hydroxy-but-2-enyl)-7-oxo-3-[2-oxo-ethyliden]-4-oxa-1-aza-bicyclo[3.2.0]heptan-2-carbonsäurebenzylester
Beispiel 9 (Isomer der Verbindung des Beispiels 8): (5R)-2-(4-Hydroxy-but-2-enyl)-7-oxo-3-[2-oxo-ethyliden]-4-oxa-1-aza-bicyclo[3.2.0]heptan-2-carbonsäurebenzylester

Analog zu dem in den Beispielen 1-3 beschriebenen Verfahren, jedoch unter Verwendung der Verbindung des Beispiels A und von 4-Vinyl-[1,3]dioxolan-2-on, wird die Verbindung des Beispiels 7 als Öl mit den untenstehenden Kenndaten

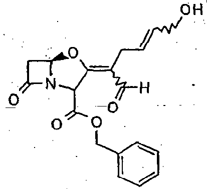

**PatAZ2574i ¹H-NMR** (DMSO-d₆, 400 MHz): δ 9,61 (s, 1 H); 7,40-7,33 (m, 5 H); 6,44 (s, 1 H); 6,00 (d, J = 2,4, 1 H); 5,54-5,39 (m, 2 H); 5,23 und 5,19 (zwei Dubletten als ABq, J = 12,4 Hz, 2 H); 4,57 (t, J = 5,5 Hz, 1 H); 3,82 (br. m, 2 H); 3,78 (dd, J = 2,9,17,0 Hz, 1 H); 3,30 (d, J = 16,8 Hz, 1 H); 2,93 (br. d, J = 4,0 Hz, 2 H);
genauso wie die Verbindung des Beispiels 8 als Öl mit den untenstehenden Kenndaten

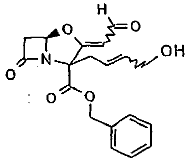

Pat **AZ2530i, ¹H-NMR** (DMSO-d₆, 400 MHz): δ 9,87 (d, J = 7,9 Hz, 1 H): 7,41-7,32 (m, 5 H); 5,90 (d, J = 2,3 Hz, 1 H); 5,76-5,70 (m, 1 H); 5,50 (dt, Jd = 15,5, Jt = 7,3 Hz, 1 H); 5,36 (d, J = 7,9 Hz, 1 H); 5,26 (d, J = 12,4 Hz, 1 H); 5,10 (d, J = 12,5 Hz, 1 H); 4,70 (t, J = 5,5 Hz, 1 H); 3,89 (t, J = 4,6 Hz, 2 H); 3,64 (dd, J = 2,8, 17,0 Hz, 1 H); 3,38 (d, J = 17,1 Hz, 1 H); 2,82 (dd, J = 7,5, 14,1 Hz, 1 H); 2,68 (dd, J = 14,4, 6,7 Hz, 1 H);
und die Verbindung des Beispiels 9 als Öl mit den untenstehenden Kenndaten

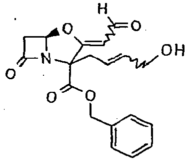

Pat**AZ2570i, ¹H-NMR,** (DMSO-d₆, 400 MHz): δ 9,84 (d, J = 7.,9 Hz, 1 H); 7,40-7,32 (m, 5 H); 5,93 (d, J = 2,5 Hz, 1 H); 5,72-5,69 (m, 2 H); 5,45 (d, J = 8,0 Hz, 1 H); 5,18 (s, 2 H); 4,70 (t, J = 5,4 Hz, 1 H); 3,84-3,80 (br. m, 2 H); 3,69 (dd, J = 2,9, 16,9 Hz, 1 H); 3,40 (d, J = 16,9 Hz, 1 H); 3,13-3,05 (m, 1 H); 2,85-2,79 (m, 1 H)
erhalten.

### Beispiele 10, 11 und 12

Beispiel 10: (5R)-3-[5-Acetoxy-1-formyl-pent-3-enyliden]-7-oxo-4-oxa-1-aza-bicyclo[3.2.0]heptan-2-carbonsäurebenzylester
Beispiel 11: (5R)-2-(4-Acetoxy-but-2-enyl)-7-oxo-3-[2-oxo-ethyliden]-4-oxa-1-aza-bicyclo[3.2.0]heptän-2-carbonsäurebenzylester
Beispiel 12 (Isomer der Verbindung des Beispiels 1.1): (5R)-2-(4-Acetoxy-but-2-enyl)-7-oxo-3-[2-oxo-ethyliden]-4-oxa-1-aza-bicyclo[3.2.0]heptan-2-carbonsäurebenzylester

Analog zu dem in den Beispielen 1-3 beschriebenen Verfahren, jedoch unter Verwendung der Verbindung des Beispiels A und von Essigsäure-4-ethoxycarbonyloxy-but-2-enylester, wird die Verbindung des Beispiels 10 als Öl mit den untenstehenden Kenndaten

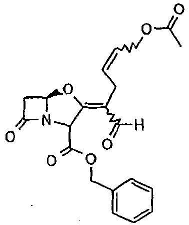

**PatAZ 02579 I, ¹H-NMR** (DMSO-d₆, 400 MHz): Mischung von Isomeren; Daten des Hauptisomers: δ 9,61 (s, 1 H); 7,45-7,23 (m, 5 H); 6,47 (s, 1 H); 6,02 (d, J = 2,3 Hz, 1 H); 5,23 & 5,19 (zwei Dubletten als ABq, J = 12,6 Hz, 2 H); 3,79 (dd, J = 2,4, 17,2 Hz, 1 H); 3,30 (d überlappend mit anderen Signalen, J = 16,8 Hz, 1 H); 2,96 (ABx m, J(AB) = 15,4, J(Ax) = J(Bx) = 5,3 Hz, 2 H); 1,98 (s, 3 H);
genauso wie die Verbindung des Beispiels 11 als Öl mit den untenstehenden Kenndaten

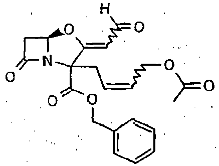

Pat**AZ2580i, ¹H-NMR** (DMSO-d₆, 400 MHz): δ 9,84 (d, J = 7,7 Hz, 1 H); 7,40-7,37 (br. s, 5 H); 5,95 (d, J = 2,0 Hz, 1 H); 5,83 (ddd, J = 5,9, 7,7, 14,8 Hz, 1 H); 5,71 (dt, Jd = 15,4, Jt = 5,7 Hz, 1 H); 5,46 (d, J = 7,9 Hz, 1 H); 5,19 (s, 2 H); 4,39 (d, J J = 5,7 Hz, 2 H); 3,70 (dd, J= 2,2, 16,9 Hz, 1 H); 3,41 (d, J = 16,8 Hz, 1 H); 3,15 (dd, J = 8,4, 14,6 Hz, 1 H); 2,85 (dd, J = 4,8, 14,4 Hz, 1 H); 1,99 (s, 3 H);
und die Verbindung des Beispiels 12 als Öl mit den untenstehenden Kenndaten

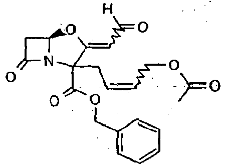

Pat**AZ 2581i ¹H-NMR** (DMSO-d₆, 400 MHz): δ 9,85 (d, J = 7,9 Hz, 1 H); 7,40-7,33 (br. s, 5 H); 5,90 (br. s, 1 H); 5,75-5,62 (m, 2 H); 5,37 (d, J = 7,9 Hz, 1 H); 5,27 & 5,11 (zwei Dubletten als ABq, J = 12,4 Hz, 2 H); 4,47 (d, J = 5,3 Hz, 2 H); 3,66 (dd, J = 2,0, 17,0 Hz, 1 H); 3,40 (d, J = 17,2 Hz, 1 H); 2,85 (dd, J = 6,3, 14,2 Hz, 1 H); 2,72 (dd, J = 6,3, 14,2 Hz, 1 H); 2,0 (s, 3 H)
erhalten.

### Beispiele 13, 14, 15

Beispiel 13: (SR)-3-((1-Formyl-4-phenyl-but-3-enyliden)-7-oxo-4-oxa-1-aza-bicyclo[3.2.0]heptan-2-carbonsäuremethylester
Beispiel 14: (5R)- 7-Oxo-3-[2-oxo-ethyliden]-2-(3-phenylallyl)-4-oxa-1-aza-bicyclo[3.2.0]heptan-2-carbonsäuremethylester
Beispiel 15 (Isomer der Verbindung des Beispiels 14): (5R)-7-Oxo-3-[2-oxo-ethyliden]-2-(3-phenylallyl)-4-oxa-1-aza-bicyclo[3.2.0]heptan-2-carbonsäuremethylester

Analog zu dem in den Beispielen 1-3 beschriebenen Verfahren, jedoch unter Verwendung der Verbindung des Beispiels B und von Carbonsäureethylester (E)-3-phenylallylester, wird die Verbindung des Beispiels 13 als Öl mit den untenstehenden Kenndaten

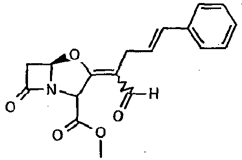

**PatAZ8256i ¹H-NMR** (DMSO-d₆, 400 MHz): δ 9,65 (s, 1 H); 7,35-7,15 (m, 5 H); 6,42 (s, 1 H); 6,33 (d, J = 16,1 Hz, 1 H); 6,21 (dt, Jd = 15,9, Jt = 6,1 Hz, 1 H); 6,04 (d, J = 2,4 Hz, 1 H); 3,78 (dd, J = 17,2, 3,0 Hz, 1 H); 3,74 (s, 3 H); 3,32 (d, J = 17,2 Hz, 1 H); 3,14 (d, J = 6,0 Hz, 2 H);
genauso wie die Verbindung des Beispiels 14 als Schaum mit den untenstehenden Kenndaten

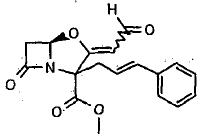

**PatAZ8273i ¹H-NMR.** (DMSO-d₆, 400 MHz): δ 9,87 (d, J = 8,0 Hz, 1 H); 7,38 -7,22 (m, 5 H); 6,62 (d, J = 15,8 Hz, 1 H); 6,33 (ddd, J = 5,7, 8,6, 15,9 Hz, 1 H); 5,98 (d, J = 2,5 Hz, 1, H); 5,50 (d, J = 8,0 Hz, 1 H); 3,72 (dd, J = 2,8, 17,0 Hz, 1 H); 3,72 (s, 3 H); 3,45 (d, J = 17,0 Hz, 1 H); 3,28 (dd, J = 8,5, 14,7 Hz, 1 H); 3,02 (ddd, J = 1,3, 5,6, 14,7 Hz, 1 H);
und die Verbindung des Beispiels 15 als Schaum mit den untenstehenden Kenndaten

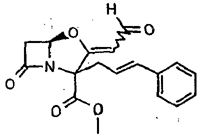

**PatAZ8271i ¹H-NMR** (DMSO-d₆, 400 MHz): δ 9,88 (d, J = 8,0 Hz, 1 H); 7,44-7,22 (m, 5 H); 6,57 (d, J = 15,8 Hz, 1 H); 6,17 (dt, Jd = 15,7, Jt = 7,5 Hz, 1 H); 5,89 (d, J = 2,2 Hz, 1 H); 5,43 (d, J = 7,9 Hz, 1 H); 3,75 (s, 3 H); 3,63 (dd, J = 2,9, 16,9 Hz, 1 H); 3,44 (d, J = 17,2 Hz, 1 H); 3,00 (dd, J = 7,9, 14,1 Hz, 1 H); 2,85 (ddd, J = 1,0, 7,3, 14,3 Hz, 1 H)
erhalten.

### Beispiele 16, und 17

Beispiel 16: (5R)-3-[1-Formyl-but-3-enyliden]-7-oxo-4-oxa-1-aza-bicyclo[3.2.0]heptan-2-carbonsäuremethylester
Beispiel 17: (5R)-2-Allyl-7-oxo-3-[2-oxo-ethyliden]-4-oxa-1-aza-bicyclo[3.2.0]heptan-2-carbonsäuremethylester

Analog zu dem in den Beispielen 1-3 beschriebenen Verfahren, jedoch unter Verwendung der Verbindung des Beispiels B und von Carbonsäureallylesterethylester, wird die Verbindung des Beispiels 16 als Öl mit den untenstehenden Kenndaten

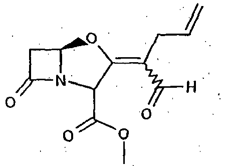

**PatAZ8277i ¹H-NMR** (DMSO-d₆, 400MHz): 9,60 (s, 1 H); 6,39 (s, 1 H); 6,00 (d, J= 2,7 Hz, 1 H), 5,81-5,71 (m, 1 H); 4,94 (dd, J = 16,6, 1,7 Hz, 1 H); 4,93 (dd, J = 10,6, 1,7 Hz, 1 H); 3,78 (dd, J = 17,2, 3,1 Hz, 1 H); 3,27 (d, J = 17,4 Hz, 1 H); 2,96 (d, J = 6,0 Hz, 2 H);
genauso wie die Verbindung des Beispiels 17 als Öl mit den untenstehenden Kenndaten

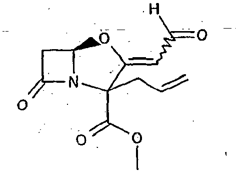

**PatAZ8278i ¹H-NMR** (DMSO-d₆, 400 MHz): Mischung von Isomeren
Daten des Hauptisomers: δ 9,85 (d, J = 7,7 Hz, 1 H); 5,45 (d, J = 7,9 Hz, 1 H); 5,22-5,18 (br. m, 2 H); 3,71 (s, 3 H); 3,42 (d, J = 17,0 Hz, 1 H); 3,10 (dd, J = 8,5, 14,7 Hz, 1 H);
Daten des Nebenisomers: δ 9,87 (d, J = 7,7 Hz, 1 H); 5,38 (d, J = 7,9 Hz, 1 H); 5,28-5,23 (br. m, 2 H); 3,72 (s, 3 H); 3,64 (dd, J = 2,9 17,0 Hz, 1 H); 3,44 (dd, J = 17,0, 0,8 Hz, 1 H); 2,67
(dd, J = 7,1, 14,1 Hz, 1 H)
erhalten.

### Beispiele 18 und 19

Beispiel 18: (5R)-3-[1-Formyl-4-phenyl-but-3-enyliden]-7-oxo-4-oxa-1-aza-bicyclo[3.2.0]heptan-2-carbonsäure-2,2-dimethylpropionyloxymethylester
Beispiel 19: (5R)-7-Oxo-3-(2-oxo-ethyliden)-2-(3-phenylallyl)-4-oxa-1-aza-bicyclo[3.2.0]heptan-2-carbonsäure-2,2-dimethylpropionyloxymethylester

Analog zu dem in den Beispielen 1-3 beschriebenen Verfahren, jedoch unter Verwendung der Verbindung des Beispiels C und von Carbonsäureethylester (E)-3-phenylallylester, wird die Verbindung des Beispiels 18 als Öl mit den untenstehenden Kenndaten

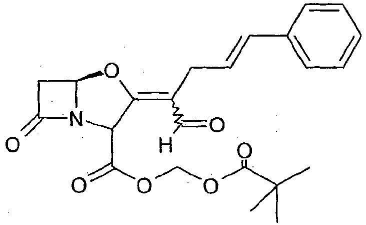

**PatAZ9488H ¹H-NMR** (DMSO-d₆, 400 MHz): δ 9,61 (s, 1 H); 7,35-7,17 (m, 5 H); 6,50 (s, 1 H); 6,34 (d, J = 15,9 Hz, 1 H); 6,18 (dt, Jd = 15,9, Jt = 6,3 Hz, 1 H); 6,02 (d, J = 2;4 Hz, 1 H); 5,82 & 5,77 (zwei Dubletten als ABq, J = 5,8 Hz, 2 H); 3,81 (dd, J = 3,1, 17,2 Hz, 1 H); 3,35 (dd, J = 17,2, 0,6 Hz, 1 H); 3,12 (dd, J = 6,4, 0,9 Hz, 2 H); 1,08 (br. s, 9 H);
genauso wie die Verbindung des Beispiels 19 als Öl mit den untenstehende Kenndaten

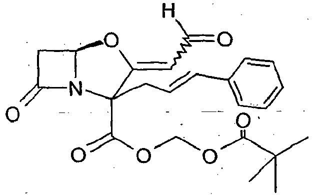

**PatAZ9487H ¹H-NMR** (DMSO-d₆, 400 MHz): Mischung von Isomeren:
Daten des Hauptisomers: δ 9,86 (d, J = 7,7 Hz, 1 H); 7,42-7,14 (mehrere Multipletten, 5 H); 6,65 (d, J = 16,1. Hz, 1 H); 6,33 (ddd, J = 5,7, 8,4, 15,9 Hz, 1 H); 5,95 (d, J = 2,2 Hz, 1 H); 5,80 & 5,78 (zwei Dubletten als ABq, J = 6,0 Hz, 2 H); 5,47 (d, J = 7,9 Hz, 1 H); 3,74 (dd, J = 3,1, 17,0 Hz, 1 H); 3,47 (d, J = 17,0 Hz, 1 H); 1,02 (s, 9 H);
Daten des Nebenisomers: δ 9,87 (d, J = 7,9 Hz, 1 H); 7,42-7,14 (mehrere Multipletten, 5 H); 6,58 (d, J = 1 15,9 Hz, 1 H); 6,15 (dt, Jd = 15,9, Jt = 7,5 Hz, 1 H); 5,93 (dd, J = 2,9, 0,7 Hz, 1 H); 5,88 & 5,84 (zwei Dubletten als ABq, J = 5,2 Hz, 2 H); 5,32 (d, J = 7,9 Hz, 1 H); 3,64 (dd, J = 3,0, 17,0 Hz, 1 H); 3,42 (d überlappend mit anderen Signalen, J = 17,0 Hz, 1 H); 1,13 (s,9H)
erhalten.

### Beispiel 20

### (5R)-3-(5-Brom-1-formyl-pent-3-enyliden)-7-oxo-4-oxa-1-aza-bicyclo[3.2.0]heptan-2-carbonsäurebenzylester

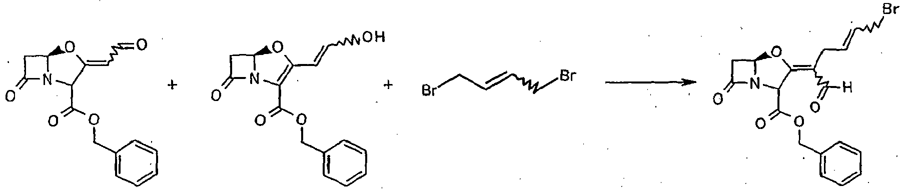

Eine Mischung aus der Verbindung des Beispiels A (30,7 g), 160 ml Dimethylformamid, K₂CO₃ (6,6 g) und 1,4-Dibrombut-2-en (20,57 g) wird bei 0-5 °C 4 Stunden lang unter Argon gerührt. Die Mischung wird zwischen 1 Liter Ethylacetat und 1 Liter Wasser, enthaltend KHSO₄ (14,5 g), aufgeteilt. Die organische Phase wird abgetrennt, mit Kochsalzlösung gewaschen, getrocknet (MgSO₄) und von den Lösungsmitteln abgezogen, um einen Rückstand zu erhalten. Der Rückstand wird unter Verwendung von Toluol / Ethylacetat (12/1) an einer SiO₂-Säule chromatographiert. Die Titelverbindung wird als Öl mit den untenstehenden Kenndaten erhalten.

**PatAZ1828k ¹H-NMR** (DMSO-d6, 400 MHz): Mischung von Isomeren; Daten des Hauptisomers: δ 9,61 (s, 1 H); 7,46-7,29 (m, 5 H); 6,48 (s, 1 H); 6,01 (d, J = 2,7 Hz, 1 H); 5,75 (dt als Quintett, Jd = 15,2, Jt = 6,0 Hz, 1 H); 5,58 (dt als Quintett, Jd = 15,0, Jt = 7,6 Hz, 1 H); 5,23 & 5,19 (zwei Dubletten als ABq, J ca. 12 Hz, 2 H); 4,06 (d, J = 7,5 Hz, 2 H); 3,79 (dd, J = 17,1, 3,0 Hz, 1 H); 3,31 (d ist unter einem H₂O-Signal vergraben); 3,04-2,91 (m, 2 H).

### Beispiele 21 und 22

Beispiel 21: (5R)-3-[5-(Benzyl-benzyloxycarbonyl-amino)-1-formyl-pent-3-enyliden]-7-oxo-4-oxa-1-aza-bicyclo[3.2.0]heptan-2-carbonsäurebenzylester
Beispiel 22: (5R)-2-[4-(Benzyl-benzyloxycarbonyl-amino)-but-2-enyl]-7-oxo-3-[2-oxo-ethyliden]-4-oxa-1-aza-bicyclo[3.2.0]heptan-2-carbonsäureben;zylester

Analog zu dem im Beispiel 20 beschriebenen Verfahren, jedoch unter Verwendung der Verbindung des Beispiels A und der Verbindung des Beispiels D, wird die Verbindung des Beispiels 21 als Öl mit den untenstehenden Kenndaten

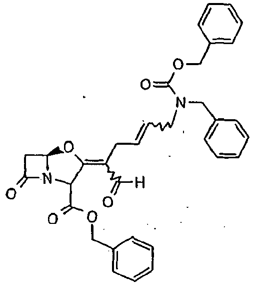

**PatAZ1834k ¹H-NMR** (DMSO-d₆, 400 MHz): δ 9,60 (s, 1 H); 7,41-7,29 (m, 15 H), 6,45 (s, 1 H); 5,97 (d, J = 2,7 Hz, 1 H); 5,52-5,09 (überlappende Multipletten, 6 H); 4,33 (s, 2 H); 3,78 (dd, J = 17,2,3,1 Hz, 1 H); 3,74-3,70 (br. d, 2 H); 3,28-3,19 (br. d, 1 H); 2,92 (d, J = 5,5 Hz, 2 H);
genauso wie die Verbindung des Beispiels 22 als Öl mit den untenstehenden Kenndaten

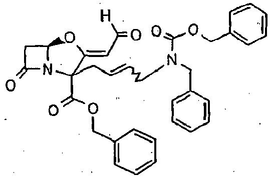

**PatAZ1835k** (DMSO-d₆, 400 MHz): Mischung von Isomeren; Daten des Hauptisomers: δ 9,84 (d, J = 7,9 Hz, 1 H); 7,40-7,14 (m, 15 H), 5,57 (dt, Jd = 15,2, Jt = 5,8 Hz, 1 H); 5,25 (d, J = 12,4 Hz, 1 H); 5,10 (s, 2 H); 5,09 (d, J = 12,1 Hz, 1 H); 4,36 (br. m, 2 H); 3,57 (br. dd, J = 17,0, 2,2 Hz, 1 H); 3,37 (br. d, 1 H); 2,86-2,59 (zwei br. Multipletten, 2 H)
erhalten.

### Beispiel 23

### (5R)-3-(5-Azido-1-formyl-pent-3-enyliden)-7-oxo-4-oxa-1-aza-bicyclo[3.2.0]heptan-2-carbonsäurebenzylester

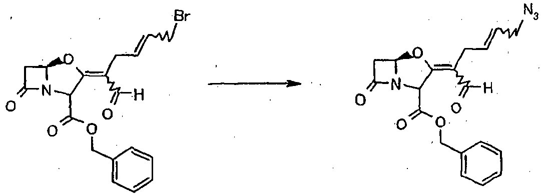

Zu einer eiskalten Mischung aus der Verbindung des Beispiels 20 (900 mg) und 15 ml Tetrahydrofuran wird unter Rühren eine Lösung von Tetrabutylammoniumazid (730 mg) in 15 ml Tetrahydrofuran auf einmal hinzugefügt, und das Rühren wird bei 0-5 °C 55 Minuten lang fortgesetzt. Die Mischung wird zwischen 350 ml Ethylacetat und 350 ml Kochsalzlösung aufgeteilt. Die organische Phase wird abgetrennt und mit 350 ml Kochsalzlösung gewaschen, getrocknet (MgSO₄) und von den Lösungsmitteln abgezogen, um eine Mischung zu erhalten, die unter Verwendung von Toluol / Ethylacetat (8/1) auf SiO₂ chromatographiert wird. Die Titelverbindung wird als Öl mit den untenstehenden Kenndaten erhalten. **PatAZ1849k ¹H-NMR** (DMSO-d₆, 400 MHz): Mischung von Isomeren; Daten des Hauptisomers: δ 9,62 (s, 1 H); 7,37-7,32 (m, 5 H); 6,47 (s, 1 H); 6,00 (d, J = 2,7 Hz, 1 H); 5,74-5,67 (m, 1 H); 5,43 (dtt, Jd = 15,2, Jt = 6,6, 1,3 Hz, 1 H); 5,26-5,16 (überlappende Multipletten, 2 H); 3,79 (dd, J = 3,0, 17,2 Hz, 1 H); 3,73 (d, J = 6,6 Hz, 2 H); 3,29 (d, teilweise unter einem H₂O-Signal vergraben, 1 H); 2,98 (d, J = 6,0 Hz, 2 H).

### Beispiele 24 und 25

Beispiel 24: (SR)-3-(1-Carboxybut-3-enyliden)-7-oxo-4-oxa-1-aza-bicyclo[3.2.0]heptan-2-carbonsäurebenzylester
Beispiel 25: (5R)-2-Allyl-3-[1-carboxymethyliden]-7-oxo-4-oxa-l-aza-bicyclo[3.2.0]heptan-2-carbonsäurebenzylester

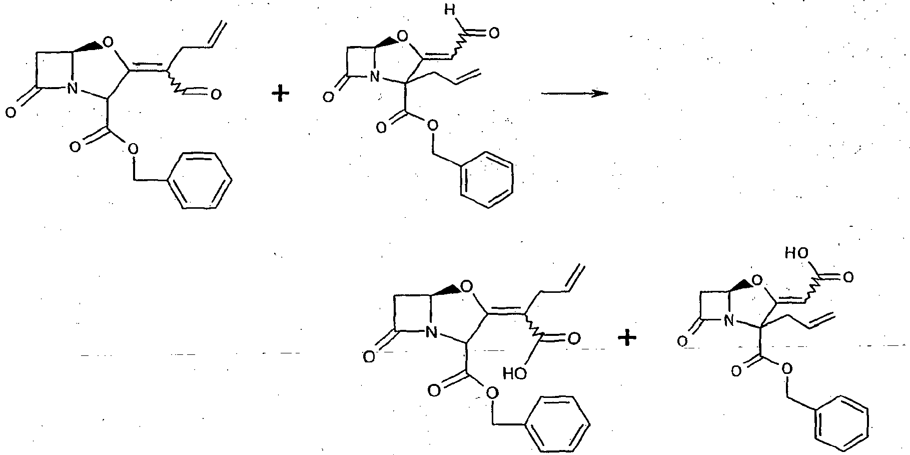

Zu einer Mischung aus der Verbindung des Beispiels 1 und der Verbindung des Beispiels 2 (37,76 g) werden 1130 ml t-Butanol, 370 ml Tetrahydrofuran und 2-Methyl-2-buten (49,5 ml) hinzugefügt. Die Mischung wird bei Raumtemperatur 15 Minuten lang gerührt, danach auf 0-5°C abgekühlt, und 1M kaltes wässriges NaH₂PO₄ (475,8 ml) wird auf einmal hinzugefügt, gefolgt von NaClO₂ (39,12 g) in 3 Teilen, und zwar in Abständen von 5 Minuten. Nach 45-minütigem Rühren bei 0-5°C wird das Eisbad entfernt und das Rühren bei Raumtemperatur fortgesetzt. Nach einer Gesamtreaktionszeit von 3,5 Stunden werden die Lösungsmittel unter Vakuum entfernt, um eine Suspension zu erhalten. 380 ml Ethylacetat und 476 ml von 1M kaltem wässrigem NaH₂PO₄ werden hinzugefügt. Die organische Phase wird abgetrennt, und die wässrige Phase wird mit 159 ml von kaltem 1M NaH₂PO₄ angesäuert und mit 380 ml Ethylacetat extrahiert. Diese Ansäuerung und Extraktion der wässrigen Phase wird nochmals wiederholt. Die kombinierten organischen Extrakte werden getrocknet (MgSO₄) und von den Lösungsmitteln abgezogen, um eine Mischung zu erhalten, die unter Verwendung von Toluol / Methyl-t-butylether (15/1, 5/1 und 1/3) an einer SiO₂-Säule chromatographiert wird. Die Verbindung des Beispiels 24 wird als Öl mit den untenstehenden Kenndaten

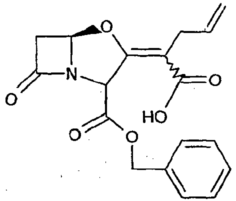

**PatAZ8288i ¹H-NMR** (DMSO-d₆, 400 MHz): δ 12,7-12,4 (br. s, 1 H); 7,41-7,31 (m, 5 H); 5,88 (d, J = 2,4, 1 H); 5,79 (ddt, Jd = 17,1, 10,1, Jt = 5,9 Hz, 1 H); 5,55 (s, 1 H); 5,16 (s, 2 H); 4,99 (dq, Jd = 17,2, Jq = 1,7 Hz, 1 H); 4,91 (dq, Jd= 10,1, Jq = 1,1 Hz, 1 H); 3,72 (dd, J =3,0, 17,1 Hz, 1 H); 3,26 (d, J = 17,0 Hz, 1, H); 3,01 (d, J = 6,0 Hz, 2 H);
erhalten, und die Verbindung des Beispiels 25 wird als Öl mit den untenstehende Kenndaten

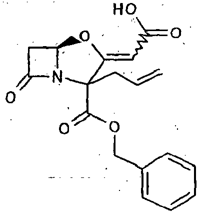

**PatAZ7208K ¹H-NMR** (DMSO-d₆, 500 MHz): Mischung von Isomeren; δ 11,99 (br. s, 1 H); 7,39-7,33 (m, 5 H); 5,94-5,86 (m, 0,6 H); 5,84 (d, J = 2,7 Hz, 0,6 H); 5,80 (d, J = 2,2 Hz, 0,4 H); 5,75-5,66 (m, 0,4 H); 5,26 (d, J = ,12,6 Hz, 0,4 H); 5,23-5,12 (überlappende Multipletten); 5,10 (s, 0,6 H); 4,96 (s, 0,4 H); 3,66 (dd, J = 17,0, 2,7 Hz, 0,6 H); 3,57 (dd, J = 17,0,2,7 Hz, 0,4 H); 3,29 (d, J = 17,0 Hz, 0,4 H); 3,28 (d, J = 17,0 Hz, 0,6 H); 3,07 (dd, J = 14,3, 8,8 Hz, 0,6 H); 2,83-2,78 (m, 1 H); 2,65 (dd, J = 14,3, 7,1 Hz, 0,4 H)
erhalten.

### Beispiel 26

### (5R)-3-[1-Carboxy-4-phenyl-but-3-enyliden]-7-oxo-4-oxa-1-aza-bicyclo[3.2.0]heptan-2-carbonsäurebenzylester

Analog zu dem in den Beispielen 24, 25 beschriebenen Verfahren, jedoch unter Verwendung der Verbindung des Beispiels 4, wird die Titelverbindung als Sirup mit den untenstehenden Kenndaten erhalten.

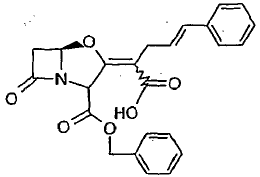

**Pat**AZ 9499h **¹H-NMR** (DMSO-d₆, 400 MHz): 8 12,62 (br. s, 1 H); 7,36-7,28 (m, 10 H); 6,37 (d, J = 16,1 Hz, 1 H); 6,23 (dt, Jd = 15,9, Jt =6,2 Hz, 1 H); 5,92 (d, J = 2,2 Hz, 1 H); 5,58 (s, 1 H); 5,19 *&* 5,15 (zwei Dubletten als ABq, J = 12,6 Hz, 2 H); 3,73 (dd, J.= 17,1, 3,0 Hz, 1 H); 3,29 (d, J = 16,8 Hz, 1 H); 3,18 (d, J = 5,5 Hz, 2 H).

### Beispiel 27

### (5R)-3-[1-Carboxymethyliden]-7-oxo-2-(3-phenylallyl)-4-oxa-1-aza-bicyclo[3.2.0]heptan-2-carbonsäurebenzylester

Analog zu dem in den Beispielen 24, 25 beschriebenen Verfahren, jedoch unter Verwendung der Verbindung des Beispiels 6, wird die Titelverbindung als Pulver mit den untenstehenden Kenndaten erhalten.

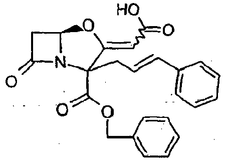

**PatAZ9497H ¹H-NMR** (DMSO-d₆, 400 MHz): δ 11,96 (br. s, 1 H); 7,39-7,23 (m, 10 H); 6,55 (d, J = 15,9 Hz, 1 H); 6,11 (dt, Jd = 15,9, Jt =7,4 Hz, 1 H); 5,81 (d, J = 2,0 Hz, 1 H); 5,29 (d, J =12,6 Hz, 1 H); 5,13 (d, J = 12,4 Hz, 1 H); 5,00 (s, 1 H); 3,58 (dd, J = 17,0, 3,1 Hz, 1 H); 3,30 (dd überlappend mit einem H₂O-Signal, J ca. 17,0, 0,7 Hz, 1 H); 2,96 (ddd, J = 0,9, 7,4, 14,2 Hz, 1 H); 2,82 (ddd, J = 0,9, 7,4, 14,4 Hz, 1 H).

### Beispiel 28

### (5R)-3-[1-Carboxymethyliden]-7-oxo-2-(3-phenylallyl)-4-oxa-1-aza-bicyclo[3.2.0]heptan-2-carbonsäurebenzylester (Isomer der Verbindung des Beispiels 27)

Analog zu dem in den Beispielen 24, 25 beschriebenen Verfahren; jedoch unter Verwendung der Verbindung des Beispiels 5, wird die Titelverbindung als Gummi mit den untenstehenden Kenndaten erhalten.

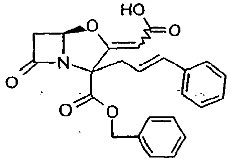

**PatAZ9495H ¹H-NMR** (DMSO-d₆, 400 MHz): δ 12,07 (br. s, 1 H); 7,34-7,23 (m, 10 H); 6,56 (d, J = 16,1 Hz, 1 H); 6,28 (ddd, J = 15,9, 8,5, 5,6 Hz, 1 H); 5,87 (d, J = 2,4 Hz, 1 H); 5,22 & 5,18 (zwei Dubletten als ABq, J = 12,6 Hz, 2 H); 5,17 (s, 1 H); 3,68 (dd, J = 17,0, 3,1 Hz, 1 H); 3,32 (d, J = 17,0 Hz, 1 H); 3,25 (dd, J = 8,5, 14,9 Hz, 1 H); 2,98 (ddd, J = 1,4, 5,6, 14,6 Hz, 1 H).

### Beispiel 29

### Kalium; 2-((5R)-2-Benzyloxycarbonyl-7-oxo-4-oxa-1-aza-bicyclo[3.2.0]hept-3-yliden)-5-phenyl-pent-4-enoat

Eine Mischung aus 109 mg Kaliumacetat und 6 ml i-Propanol wird tropfenweise zu einer Mischung aus 468 mg der Verbindung des Beispiels 26 und 6 ml i-Propanol hinzugefügt. Die Mischung wird 15 Minuten lang gerührt, und die Lösungsmittel werden unter Vakuum entfernt, um einen Rückstand zu erhalten. Der Rückstand wird mit 5 ml i-Propanol trituriert. Ein Präzipitat, das sich gebildet hat, wird filtriert und getrocknet. Die Titelverbindung wird als Pulver mit den untenstehenden Kenndaten erhalten.

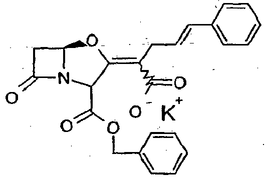

**PatAZ8241i ¹H-NMR** (DMSO-d₆, 500 MHz): δ 7,39-7,25 (m, 10 H); 6,34-6,25 (ABx-Multiplett, J(AB) = 16,0, J(Bx) = 5,0 Hz, 2 H); 5,65 (s, 1 H); 5,61 (d, J = 2,3 Hz, 1 H); 5,09 (s, 2 H); 3,57 (dd, J = 17,0, 2,8.Hz, 1 H); 3,17 (dd, J = 14,2, 4,6 Hz, 1 H); 3,10 (dd, J = 14,0, 4,8 Hz, 1 H); 3,05 (d, J = 16,5 Hz, 1 H).

### Beispiel 30

### (5R)-3-(5-Brom-1-carboxypent-3-enyliden)-7-oxo-4-oxa-1-aza-bicyclo[3.2.0]heptan-2-carbonsäurebenzylester

Analog zu dem in den Beispielen 24, 25 beschriebenen Verfahren, jedoch unter Verwendung der Verbindung des Beispiels 20, wird die Titelverbindung als Öl mit den untenstehenden Kenndaten erhalten.

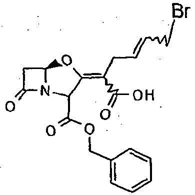

**PatAZ7207k ¹H**-**NMR** (DMSO-d₆, 400 MHz): Mischung von Isomeren; Daten des Hauptisomers: δ 7,43-7,31 (m, 5 H); 5,89 (d, J = 2,4 Hz, 1 H); 5,82 (dt, Jd = 15,0, Jt = 6,0 Hz, 1 H); 5,65 (dt als Quintett, Jd = 15,2, Jt = 7,5 Hz, 1 H); 5,54 (s, 1 H); 5,16 (s, 2 H); 4,07 (d, J = 7,3 Hz, 2 H); 3,73 (dd, J = 2,9, 17,0 Hz, 1 H); 3,27 (d, J = 17,2 Hz, 1 H), 3,02 (d, J = 5,7 Hz, 2 H).

### Beispiel 31

### (5R)-3-(5-Azido-1-carboxypent-3-enyliden)-7-oxo-4-oxa-1-aza-bicyclo[3.2.0]heptan-2-carbonsäurebenzylester

Analog zu dem in den Beispielen 24, 25 beschriebenen Verfahren, jedoch unter Verwendung der Verbindung des Beispiels 23, wird die Titelverbindung als Öl mit den untenstehende Kenndaten erhalten.

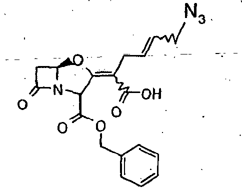

**PatAZ7202k ¹H-NMR** (DMSO-d₆, 500 MHz): Mischung von Isomeren; Daten des Hauptisomers: δ 12,7-12,0 (br. s); 7,5-7,3 (m, 5 H); 5,88 (d, J = 2,6 Hz, 1 H); 5,84-5,74 (m, 1 H); 5,54 (s, 1 H); 5,53-5,46 (m, 1 H); 5,15 (s, 2 H); 3,26 (d, J = 17,2 Hz, 1 H); 3,04 (d, J = 6,0 Hz, 2 H).

### Beispiel 32:

### (5R)-3-(1-Carboxybut-3-enyliden)-7-oxo-4-oxa-1-aza-bicyclo[3.2.0]heptan-2-carbonsäuremethylester

Analog zu dem in den Beispielen 24, 25 beschriebenen Verfahren, jedoch unter Verwendung der Verbindung des Beispiels 16, wird die Titelverbindung als Öl mit den untenstehenden Kenndaten erhalten.

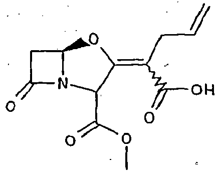

**PatAZ8283i ¹H-NMR** (DMSO-d₆, 400 MHz): δ 12,43 (br. s, 1 H); 5,87 (d, J = 2,7 Hz, 1 H); 5,81 (ddt, Jd = 17,2, 10,1, Jt = 5,7 Hz, 1 H); 5,49 (s, 1 H); 5,01 (dd, J = 17,2, 1,8 Hz, 1 H); 4,95 (dd, J = 10,1, 1,5 Hz, 1 H); 3,71 (dd, J = 17,1,3,0 Hz, 1 H); 3,67 (s, 3 H); 3;25.(d, J = 17,0 Hz, 1 H); 3,01 (d, J = 5,7 Hz, 2 H).

### Beispiel 33:

### (5R)-2-Allyl-3-carboxymethylen-7-oxo-4-oxa-1-aza-bicyclo[3.2.0]heptan-2-carbonsauremethylester

Analog zu dem in den Beispielen 24, 25 beschriebenen Verfahren, jedoch unter Verwendung der Verbindung des Beispiels 17, wird die Titelverbindung als Schaum mit den untenstelienden Kenndaten erhalten.

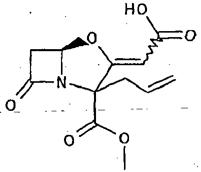

**PatAZ8297i ¹H-NMR** (DMSO-d₆, 400 MHz): Mischung von Isomeren; δ 12,00 (br. s); 5,84 (d, J = 2,7 Hz, 1 H, Hauptisomer); 5,79 (d, J = 2,2 Hz, 1 H, Nebenisomer); 5,09 (s, 1 H, groß); 4,95 (s, 1 H, klein); 3,71 (s, 3 H, klein); 3,70 (s, 3 H, groß); 3,66 (dd, J = 2,9, 17,0 Hz, 1 H, groß); 3,56 (dd, J = 2,9, 17,0 Hz, 1 H, klein); 3,33 (d überlappend mit einem H₂O-Signal); 3,28 (d, J = 17,0 Hz, 1 H, groß); 3,04 (dd, J = 8,6, 14,6 Hz, 1 H, groß); 2,62 (dd, J = 7,1, 14,3 Hz, 1 H, klein).

### Beispiel 34

### (SR)-3-(1-Carboxy-4-phenyl-but-3-enyliden)-7-oxo-4-oxa-1-aza-bicyclo[3.2.0]heptan-2-carbonsäuremethylester

Analog zu dem in den Beispielen 24, 25 beschriebenen Verfahren, jedoch unter Verwendung der Verbindung des Beispiels 13, wird die Titelverbindung als Öl mit den untenstehenden Kenndaten erhalten.

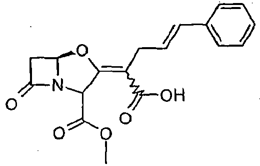

**PatAZ8260i ¹H-NMR** (DMSO-d₆, 400 MHz): δ 12,54 (br. s, 1 H); 7,37-7,14 (m, 5 H); 6,39 (d, J = 16,1 Hz, 1 H); 6,26 (dt, Jd = 15,9, Jt =6,0 Hz, 1 H); 5,91 (d, J = 2,7 Hz, 1 H); 5,53 (s, 1 H); 3,74 (dd, J = 17,2, 3,1 Hz, 1 H); 3,69 (s, 3 H); 3,29 (d, J = 17,2 Hz, 1 H); 3,18 (d, J = 5,7 Hz, 2 H).

### Beispiel 35

### (5R)-3-[1-Carboxymethyliden]-7-oxo-2-(3-phenylallyl)-4-oxa-1-aza-bicyclo[3 2.0]heptan-2-carbonsäuremethylester

Analog zu dem in den Beispielen 24, 25 beschriebenen Verfahren, jedoch unter Verwendung der Verbindung des Beispiels 15, wird die Titelverbindung als Schaum mit den untenstehenden Kenndaten erhalten.

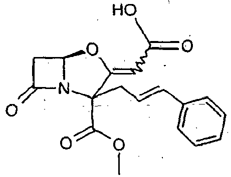

**PatAZ8280i ¹H-NMR** (DMSO-d₆, 400 MHz): δ 11,97 (br. s, 1 H); 7,41-7,23 (m, 5 H); 6,56 (d, J = 15,9 Hz, 1 H); 6,13 (dt, Jd = 15,9, Jt = 7,3 Hz, 1 H); 5,78 (d, J = 2,2 Hz, 1 H); 5,01 (s, 1 H); 3,73 (s, 3 H); 3,56 (dd, J = 2,9,17,0 Hz, 1 H); 3,33 (d, J = 16,3 Hz, 1 H); 2,96 (dd, J = 7,3, 15,0 Hz, 1 H); 2,79 (dd, J = 7,3, 15,0 Hz, 1 H).

### Beispiel 36

### (5R)-3-[1-Carboxymethyliden]-7-oxo-2-(3-phenylallyl)-4-oxa-1-aza-bicyclo[3.2.0]heptan-2-carbonsäuremethylester (Isomer der Verbindung des Beispiels 35)

Analog zu dem in den Beispielen 24, 25 beschriebenen Verfahren, jedoch unter Verwendung der Verbindung des Beispiels 14, wird die Titelverbindung als Schaum mit den untenstehenden Kenndaten erhalten.

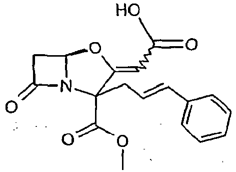

**PatAZ8262i ¹H-NMR** (DMSO-d₆, 400 MHz): δ 12,05 (br. s, 1 H); 7,37-7,23 (m, 5 H); 6,62 (d, J = 15,9 Hz, 1 H); 6,32 (ddd, J= 15,9, 8,4, 5,5 Hz, 1 H); 5,87 (d, J = 2,7 Hz, 1 H); 5,15 (s, 1 H); 3,71 (s, 3 H); 3,68 (dd, J = 2,9, 17,0 Hz, 1 H); 3,32 (d, J = 17,0 Hz, 1 H); 3,22 (dd, J = 8,6, 14,8 Hz, 1 H); 2,97 (ddd, J = 1,3, 5,5, 14,6 Hz, 1 H).

### Beispiel 37

### (5R)-3-(1-Carboxy-4-phenyl-but-3-enyliden)-7-oxo-4-oxa-1-aza-bicyclo[3.2.0]heptan-2-carbonsäure-2,2-dimethylpropionyloxymethylester

Analog zu dem in den Beispielen 24, 25 beschriebenen Verfahren, jedoch unter Verwendung der Verbindung des Beispiels 18, wird die Titelverbindung als Pulver mit den untenstehenden Kenndaten erhalten.

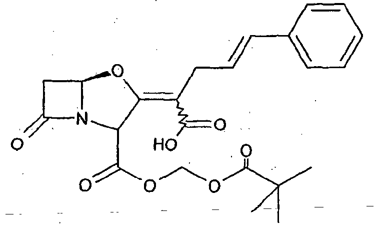

**PatAZ9496H ¹H-NMR** (DMSO-d6, 400 MHz): δ 12,50 (br. s, 1 H); 7,36 (d, J = 7,1 Hz, 2 H); 7,29 (t, J = 7,6 Hz, 2 H); 7,19 (tt, J = 7,3, 1,6 Hz, 1 H); 6,38 (d, J = 15,9 Hz, 1 H); 6,24 (dt, Jd = 15,9, Jt = 6,0 Hz, 1 H); 5,88 (d, J = 2,4 Hz, 1 H); 5,79 & 5,73 (zwei Dubletten als ABq, J = 5,8 Hz, 2 H); 5,54 (s, 1 H); 3,75 (dd, J = 17,2, 3,1 Hz, 1H); 3,31 (d überlappend mit anderen Signalen, J ca. 17,0 Hz, 1 H); 3,17 (d, J = 5,7 Hz, 2 H); 1,11 (br. s, 9 H).

### Beispiel 38, 38a

Beispiel 38: (5R)-3-[1-Hydroxymethylbut-3-en-(E)-yliden]-7-oxo-4-oxa-1-aza-bicyclo[3.2.0]heptan-2-carbonsäurebenzylester
Beispiel 38a: (5R)-3-[1-Hydroxymethylbut-3-en-(Z)-yliden]-7-oxo-4-oxa-1-aza-bicyclo[3.2.0]heptan-2-carbonsäurebenzylester

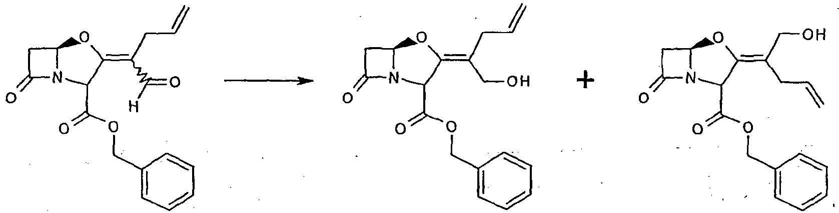

Zu einer eiskalten Mischung aus der Verbindung des Beispiels 1 (7,5 g), 300 ml Tetrahydrofuran und 100 ml Phosphatpuffer (p^{H} 7) wird eine Lösung von NaBH₄ (693 mg) in 100 ml Wasser in 4 gleichen Teilen in Zeitabständen von 10 Minuten unter Rühren hinzugefügt. Nach einer Gesamtreaktionszeit von 40 Minuten wird die Mischung zwischen 1200 ml Ethylacetat und 2,41 Wasser und 960 ml Kochsalzlösung aufgeteilt. Die wässrige Phase wird zweimal extrahiert, und zwar jeweils mit 600 ml Ethylacetat. Die organischen Extrakte werden kombiniert, getrocknet (MgSO₄) und von den Lösungsmitteln abgezogen, um ein Öl zu ergeben, das unter Verwendung von Toluol / *t*-Butylmethylether = 7/1 an einer SiO₂-Säule chromatographiert wird. Die Verbindung des Beispiels 38 wird als Öl mit den untenstehenden Kenndaten

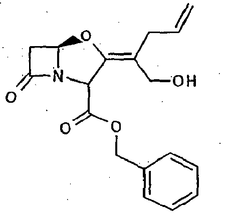

**PatAZ9457h ¹H-NMR** (DMSO-d₆, 400 MHz): δ 7,46-7,32 (m, 5 H); 5,78-5,70 (m, 1 H); 5,67 (d, J = 2,7 Hz, 1 H); 5,46 (s, 1 H); 5,17 (s, 2 H); 4,97 (dd, J = 15,0, 1,8 Hz, 1 H); 4,93 (dd, J = 7,7, 1,8 Hz, 1 H); 4,68 (dd, J = 5,0, 6,1 Hz, 1 H); 3,93 (dd, J = 4,7, 12,9 Hz, 1 H); 3,72 (dd, J = 6,2, 13,0 Hz, 1 H); 3,61 (dd, J = 17,0, 2,9 Hz, 1 H); 3,05 (d, J = 16,8 Hz, 1 H); 2,87 (dd, J = 14,8, 6,0 Hz, 1 H); 2,80 (dd, J = 14,8, 6,7 Hz, 1 H),
erhalten, und die Verbindung des Beispiels 38a wird als Öl mit den untenstehenden Kenndaten

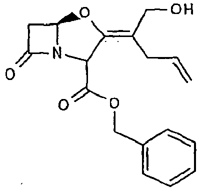

**PatAZ7225k ¹H-NMR** (DMSO-d₆, 500 MHz): δ 7,40-7,35 (m, 5 H); 5,70 (d, J = 2,7 Hz, 1H); 5,63-5,53 (m, 1H); 5,33 (s, 1H); 5,18 und 5,14 (2 d als ABq, J = 12,5 Hz, 2H); 4,91 (d, J = 18,1 Hz, 1H); 4,91 (d, J = 9,9 Hz, 1H); 4,60 (dd, J = 5,5,4,9 Hz, 1H); 4,10 (dd, J = 4,9, 12,1 Hz, 1H); 3,86 (dd, J = 6,0, 12,1 Hz, 1H); 3,62 (dd, J = 17,0, 2,9 Hz, 1H); 3,09 (d, J = 17,0 Hz, 1H); 2,80 (dd, J = 15,5, 7,1 Hz, 1H); 2,73 (dd, J = 15,4, 5,5 Hz, 1H)
erhalten.

### Beispiel 39

### (5R)-3-(1-Hydroxymethyl-4-phenylbut-3-enyliden)-7-oxo-4-oxa-1-aza-bicyclo[3.2.0]heptan-2-carbonsäurebenzylester

Analog zu dem in den Beispielen 38, 38a beschriebenen Verfahren, jedoch unter Verwendung der Verbindung des Beispiels 4, wird die Titelverbindung als Öl mit den untenstehenden Kenndaten erhalten.

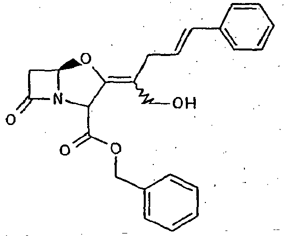

**PatAZ8220i ¹H-NMR** (DMSO-d₆, 400 MHz): δ 7,36-7,27 (m, 10 H); 6,37 (d, J = 15,9 Hz, 1 H); 6,20 (dt, Jd = 15,9, Jt = 6,6 Hz, 1 H); 5,71 (d, J = 2,4 Hz, 1 H); 5,50 (s, 1 H); 5,17 (s, 2 H); 4,73 (dd, J = 6,2,4,9 Hz, 1 H); 3,97 (dd, J = 13,0, 4,9 Hz, 1 H); 3,78 (dd, J = 13,0, 6,2 Hz, 1 H); 3,63 (dd, J = 2,9, 17,0 Hz, 1 H); 3,08 (dd, J =17,0, 0,5 Hz, 1 H); 3,03-2,96 (m, 2 H).

### Beispiel 40

### (5R)-2-Allyl-3-[2-hydroxyethyliden]-7-oxo-4-oxa-1-aza-bicyclo[3.2.0]heptan-2-carbonsäurebenzylester

Analog zu dem in den Beispielen 38, 38a beschriebenen Verfahren, jedoch unter Verwendung der Verbindung des Beispiels 2, wird die Titelverbindung als Öl mit den untenstehenden Kenndaten erhalten.

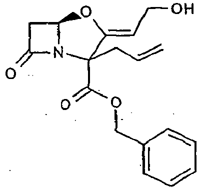

**PatAZ2522i ¹H-NMR** (DMSO-d₆, 400 MHz): Mischung von Isomeren: δ 7,43-7,31 (m); 5,97-5,86 (m, 1 H); 5,79-5,69 (m, 0,5 H); 5,58 (d, J = 2,7 Hz, 1 H); 5,47 (d, J = 2,4 Hz, 0,5 H); 5,23 (d, J = 12,8 Hz, 0,5 H); 5,20-5,10 (überlappende Multipletten, 5 H); 5,05 (d, J = 12,6 Hz, 0,5 H); 4,78 (t, J = 6,7 Hz, 1 H); 4,65 (t, J = 5,6 Hz); 4,61 (t, J = 6,9 Hz); 4,09-3,93 (überlappende Multipletten, 3 H); 3,55 (dd, J = 2,9, 16,8 Hz, 1 H); 3,41 (dd, J = 2,9, 16,8 Hz, 0,5 H); 3,10 (d, J = 16,5 Hz, 0,5 H); 3,09 (d, J = 16,8 Hz, 1 H); 3,00 (dd, J = 8,6, 14,6 Hz, 1 H); 2,77-2,71 (überlappende Multipletten, 1,5 H); 2,54-2,50 (m überlappend mit DMSO).

### Beispiele 41 und 42

Beispiel 41: (5R)-3-(5-Iodomethyl-2-oxo-dihydrofuran-3-yliden)-7-oxo-4-oxa-1-aza-bicyclo[3.2.0]heptan-2-carbonsäurebenzylester
Beispiel 42: (5R)-3-(5-Iodomethyl-2-oxo-dihydrofuran-3-yliden)-7-oxo-4-oxa-1-aza-bicyclo[3.2.0]heptan-2-carbonsäurebenzylester (Isomer der Verbindung des Beispiels 41)

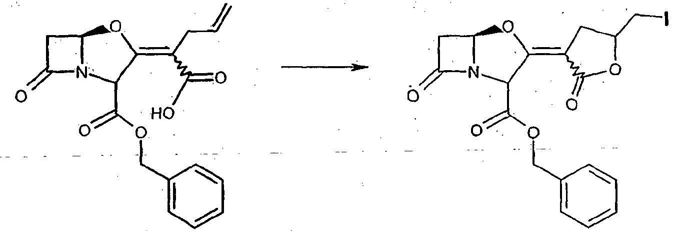

Eine Mischung aus der Verbindung des Beispiels 24 (1,63 g), 83 ml Tetrahydrofuran, Pyridin (0,765 ml) und Iod (2,41 g) wird bei Raumtemperatur gerührt. An den Zeitpunkten nach 50 Minuten und 2 h 40 min werden jeweils weitere Mengen an Pyridin (0,115 ml) und an Iod (0,362 g) hinzugefügt. Nach einer Gesamtreaktionszeit von 3 h 40 min werden die Lösungsmittel unter Vakuum entfernt, und der Rückstand wird in 330 ml Ethylacetat aufgenommen und mit wässrigem 5%igem Natriumthiosulfat.5H₂O, kaltem wässrigem 0,1%igem NaHSO₄, kaltem wässrigem 0,5%igem NaHCO₃ und danach Kochsalzlösung gewaschen. Der organische Extrakt wird getrocknet (MgSO₄) und von den Lösungsmitteln abgezogen, um einen Gummi zu erhalten, der unter Verwendung von Toluol /t-Butylmethylether (92/8) an einer SiO₂-Säule zweimal chromatographiert wird. Die Verbindung des Beispiels 41 wird als Gummi mit den untenstehenden Kenndaten 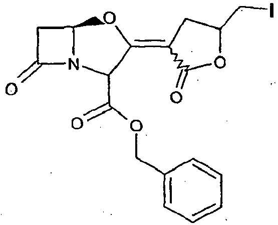 Retentionsfaktor (SiO2-tlc-Platte, Toluol/t-Butylmethylether = 3/1) = 0,65;

**PatAZ1889k** weniger polares Isomer **¹H-NMR** (DMSO-d₆, 500 MHz): δ 7,41-7,33 (m, 5 H); 5,99 (d, J = 2,7 Hz, 1 H); 5,55 (s, 1 H); 5,21 & 5,17 (zwei Dubletten als ABq, J = 12,7 Hz, 2 H); 4,65 (m, 1 H); 3,77 (dd, J = 3,0, 17,0 Hz, 1 H); 3,53 & 3,49 (zwei überlappende Multipletten, 2 H); 3,36 (d, J = 17,4 Hz, 1 H); 3,10 (ddd, J = 1,3, 8,7, 16,7 Hz, 1 H); 2,60 (ddd, J = 1,3, 5,4 16,7 Hz, 1 H);
erhalten, und die Verbindung des Beispiels 42 wird als Gummi mit den untenstehenden Kenndaten 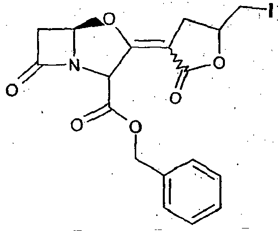 Retentionsfaktor (SiO2-tlc-Platte, Toluol/t-Butylmethylether = 3/1) = 0,56;

**PatAZ1890k mehr polares Isomer ¹H-NMR** (DMSO-d₆, 500 MHz): δ 7,40-7,33 (m, 5 H); 6,01 (d, J = 2,7 Hz, 1 H); 5,55 (s, 1 H); 5,19 & 5,16 (zwei Dubletten als sehr nahe ABq, J = 12,7 Hz, 2 H); 4,59 (m, 1 H); 3,77 (dd, J = 3,4, 17,4 Hz, 1 H); 3,52 (dd, J = 4,7, 10,7 Hz, 1 H); 3,46 (dd, J = 4,7, 10,7 Hz, 1 H); 3,34 (d, J = 17,4 Hz, 1 H); 3,08 (ddd, J = 1,3, 8,0, 16,7 Hz, 1 H); 2,56 (ddd, J =1,3, 5,4, 16,7 Hz, 1 H)
erhalten.

### Beispiele 43 und 44

Beispiel 43: (5R)-3-(5-Iodomethyl-2-oxo-dihydrofuran-3-yliden)-7-oxo-4-oxa-1-aza-bicyclo[3.2.0]heptan-2-carbonsäuremethylester
Beispiel 44: (5R)-3-(5-Iodomethyl-2-oxo-dihydrofuran-3-yliden)-7-oxo-4-oxa-1-aza-bicyclo[3.2.0]heptan-2-carbonsäuremethylester (Isomer der Verbindung des Beispiels 43)

Analog zu dem in den Beispielen 41, 42 beschriebenen Verfahren, jedoch unter Verwendung der Verbindung des Beispiels 32, wird die Verbindung des Beispiels 43 als Schaum mit den untenstehenden Kenndaten

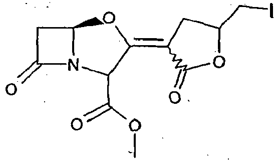

Elutionsreihenfolge bei der SiO2-Chromatographie unter Verwendung von Toluol /t-Butylmethylether: Die Verbindung des Beispiels 43 wird vor der Verbindung des Beispiels 44 eluiert.

**PatAZ1822k weniger polares Isomer ¹H-NMR** (DMSO-d₆, 400 MHz): δ 6,00 (d, J = 2;9 Hz, 1 H); 5,49 (s, 1 H); 4,59 (m, 1 H); 3,76 (dd, J = 17,2, 2,9 Hz, 1 H); 3,69 (s, 3 H); 3,56 (dd, J = 10,8,4,9 Hz, 1 H); 3,52 (dd, J = 10,8, 4,6 Hz, 1 H); 3,33 (d überlappend mit einem H₂O-Signal, 1 H); 3,08 (ddd, J = 1,1, 8,3, 16,6 Hz, 1 H); 2,57 (ddd, J = 1,5, 5,5, 16,8 Hz, 1 H);
erhalten, und die Verbindung des Beispiels 44 wird als Schaum mit den untenstehende Kenndaten

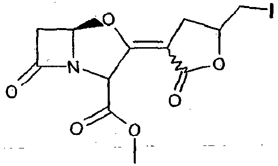

Elutionsreihenfolge bei der SiO2-Chromatographie unter Verwendung von Toluol /t-Butylmethylether: Die Verbindung des Beispiels 44 wird nach der Verbindung des Beispiels 43 eluiert.

Pat **AZ1823k** mehr polares Isomer **¹H-NMR** (DMSO-d₆, 400 MHz): δ 5,99 (d, J = 2,7 Hz, 1 H); 5,49 (s, 1 H); 4,66 (m, 1 H); 3,77 (dd, J = 17,2, 3,1 Hz, 1 H); 3,69 (s, 3 H); 3,52 (dd, J = 14,6, 5,0 Hz, 1 H); 3,49 (dd, J = 14,6, 5,0 Hz, 1 H); 3,35 (d, J = 17,2 Hz, 1 H); 3,11 (ddd, J = 1,6, 8,5, 16,8 Hz, 1 H); 2,59 (ddd, J= 1,5, 5,1, 16,8 Hz, 1 H)
erhalten.

### Beispiel 45

### (5R)-3-(5-Methyl-2-oxo-dihydrofuran-3-yliden)-7-oxo-4-oxa-1-aza-bicyclo[3.2.0]heptan-2-carbonsäurebenzylester

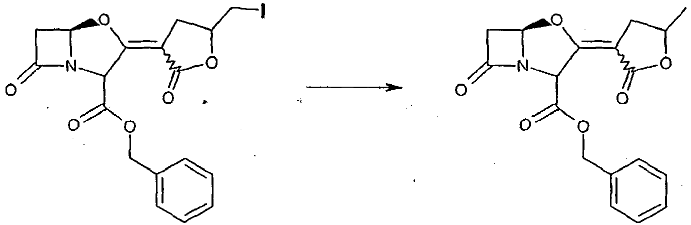

Eine Mischung von 1800 mg 10%igem Pd/C und 390 ml Ethylacetat wird bei Raumtemperatur unter einer Wasserstoffatmosphäre 30 Minuten lang gerührt. Mit einer Spritze wird eine Lösung der Verbindung des Beispiels 41 (800 mg) in 10 ml Ethylacetat eingebracht, und das Rühren wird unter der Wasserstoffatmosphäre fortgesetzt. An den Zeitpunkten nach 1 h 20 min, 2 h 40 min, 4 h und 5 h 35 min werden weitere Mengen von jeweils 1800 mg 10%igem Pd/C hinzugefügt. Nach einer Gesamtreaktionszeit von 6 h 50 min wird die Mischung durch Celit filtriert und von den Lösungsmitteln abgezogen, um einen Rückstand zu erhalten, der unter Verwendung von Toluol / t-Butylmethylether (17/1) an SiO₂ dreimal chromatographiert wird. Die Titelverbindung wird als Schaum mit den untenstehenden Kenndaten erhalten.

**PatAZ1815k** weniger polare 1-Reihen **¹H-NMR** (DMSO-d₆, 400 MHz): δ 7,40-7,34 (m, 5 H); 5,96 (d, J = 2,7 Hz, 1 H); 5,57 (t, J = 1,6 Hz, 1 H); 5,18 (ABq als Pseudo-t, J = 13,3 Hz, 2 H); 4,76-4,67 (m, 1 H); 3,76 (dd, J = 3,0, 17,0 Hz, 1 H); 3,33 (d überlappend mit einem H₂O-Signal, 1 H); 3,10 (ddd, J = 1,7, 8,0, 16,3 Hz, 1 H); 2,50 (ddd vergraben unter DMSO, J = 1,8, 5,7, 16,5 Hz, 1 H); 1,32 (d, J = 6,2 Hz, 3 H).

### Beispiel 46

### (5R)-3-(5-Methyl-2-oxo-dihydrofuran-3-yliden)-7-oxo-4-oxa-1-aza-bicyclo[3.2.0]heptan-2-carbonsäurebenzylester (Isomer der Verbindung des Beispiels 45)

Analog zu dem im Beispiel 45 beschriebenen Verfahren, jedoch unter Verwendung der Verbindung des Beispiels 42, wird die Titelverbindung als Öl mit den untenstehende Kenndaten erhalten.

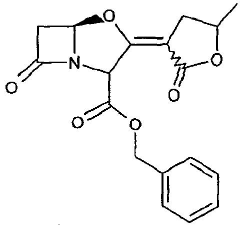

**PatAZ1813k** mehr polare Iodidreihen **¹H-NMR** (DMSO-d₆, 400 MHz): δ 7,40-7,33 (m, 5 H); 5,97 (d, J = 2,7 Hz, 1 H); 5,56 (t, J = 1,1 Hz, 1 H); 5,20 & 5,16 (zwei Dubletten als ABq, J = 12,8 Hz, 2 H); 4,75-4,67 (m, 1 H); 3,76 (dd, J = 3,0, 17,0 Hz, 1 H); 3,33 (überlappend d, 1 H); 3,09 (ddd, J =1,5, 7,9, 16,3 Hz, 1 H); 2,48 (m vergraben unter DMSO); 1,29 (d, J = 6,2 Hz, 3 H).

### Beispiel 47

### (5R)-3-(5-Azidomethyl-2-oxo-dihydrofuran-3-yliden)-7-oxo-4-oxa-1-aza-bicyclo[3.2.0]heptan-2-carbonsäurebenzylester

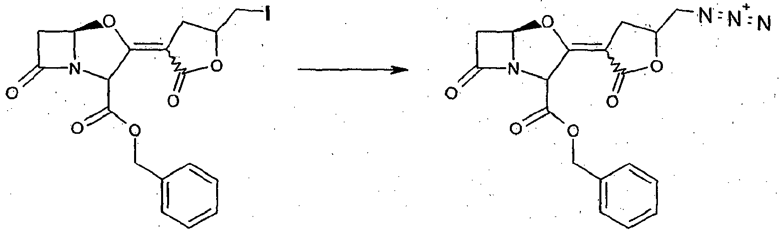

Eine Mischung aus der Verbindung des Beispiels 41 (500 mg), 50 ml Tetrahydrofuran und Tetrabutylammoniumazid (364 mg) wird bei Raumtemperatur 30 Minuten lang gerührt. Die Mischung wird zwischen Ethylacetat und Kochsalzlösung aufgeteilt. Die organische Phase wird abgetrennt, mit Kochsalzlösung gewaschen, getrocknet (MgSO₄) und von den Lösungsmitteln abgezogen. Der Rückstand wird unter Verwendung von Toluol / t-Butyl-methylether (12/1) an einer SiO₂-Säule chromatographiert. Die Titelverbindung wird als Öl mit den untenstehenden Kenndaten erhalten.

**PatAZ1876k weniger polare I-Reihen ¹H-NMR** (DMSO-d₆, 400 MHz): δ 7,45-7,31 (m, 5 H); 5,99 (d, J = 2,7 Hz, 1 H); 5,58 (t, J = 1,1 Hz, 1 H); 5,21 & 5,16 (zwei Dubletten als ABq, J = 12,7 Hz, 2 H); 4,84-4,78 (symmetrische 9-Linie m, 1 H); 3,77 (dd, J = 3,0,17,1 Hz, 1 H); 3,64 (dd, J = 3,4, 13,3 Hz, 1 H); 3,56 (dd, J = 6,0, 13,5 Hz, 1 H); 3,35 (d überlappend mit einem H₂O-Signal); 3,06 (ddd, J = 1,5, 8,6, 16,8 Hz, 1 H); 2,64 (ddd, J = 1,5, 5,3, 16,8 Hz, 1 H).

### Beispiel 48

### (5R)-3-(5-Azidomethyl-2-oxo-dihydrofuran-3-yliden)-7-oxo-4-oxa-1-aza-bicyclo[3.2.0]heptan-2-carbonsäurebenzylester (Isomer der Verbindung des Beispiels 47)

Analog zu dem im Beispiel 47 beschriebenen Erfahren, jedoch unter Verwendung der Verbindung des Beispiels 42, wird die Titelverbindung als Schaum mit den untenstehenden Kenndaten erhalten.

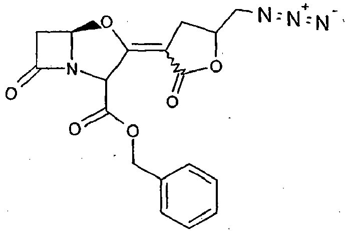

**PatAZ1839k mehr polare 1-Reihen** ¹H-NMR (DMSO-d₆, 400 MHz): δ 7,46-7,30 (m, 5 H); 6,00 (d, J = 2,4 Hz, 1 H); 5,57 (t, J = 1,7 Hz, 1 H); 5,18 (s, 2 H); 4,83-4,78 (symmetrische 9-Linie m, 1 H); 3,77 (dd, J = 3,1, 17,2 Hz, 1 H); 3,67 (dd, J = 3,1, 13,5 Hz, 1 H); 3,49 (dd, J = 5,6, 13,6 Hz, 1 H); 3,34 (d, J = 17,0 Hz, 1 H); 3,03 (ddd, J = 1,5, 8,6, 16,5 Hz, 1 H); 2,65 (ddd, J = 1,7, 5,5, 16,8 Hz, 1 H).

### Beispiel 49

### (5R)-3-(5-Methyl-2-oxo-dihydrofuran-3-yliden)-7-oxo-4-oxa-1-aza-bicyclo[3.2.0]heptan-2-carbonsäure

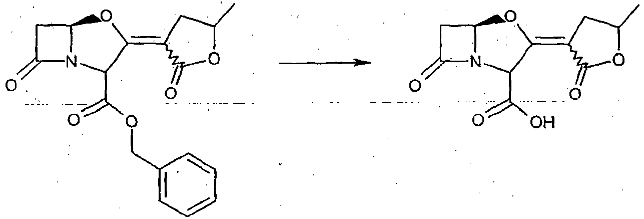

Eine Mischung von 750 mg 10%igem Pd/C und 140 ml Ethylacetat wird bei 0-5°C unter einer Wasserstoffatmosphäre 30 Minuten lang gerührt. Mit einer Spritze wird eine Lösung der Verbindung des Beispiels 45 (448 mg) in 10 ml Ethylacetat eingebracht, und das Rühren wird unter der Wasserstoffatmosphäre bei 0-5°C für 1 h 15 min fortgesetzt. Die Mischung wird durch Celit filtriert und vom Lösungsmittel abgezogen. Die Titelverbindung wird als Schaum mit den untenstehenden Kenndaten erhalten.

**PatAZ1818k weniger polare I-Reilien ¹H-NMR** (DMSO-d₆, 400 MHz): δ 5,92 (d, J = 2,7 Hz, 1 H); 5,41 (s, 1 H); 4,74-4,66 (m, 1 H); 3,72 (dd, J = 2,9, 17,0 Hz, 1 H); 3,27 (d überlappend mit H₂O); 3,08 (ddd, J = 1,3, 8,2, 16,3 Hz, 1 H); 2,48 (m teilweise vergraben unter DMSO); 1,31 (d, J = 6,4 Hz, 3 H).

### Beispiel 50

### (5R)-3-(5-Methyl-2-oxo-dihydrofuran-3-yliden)-7-oxo-4-oxa-1-aza-bicyclo[3.2.0]heptan-2-carbonsäure (Isomer der Verbindung des Beispiels 49)

Analog zu dem im Beispiel 49 beschriebenen Verfahren, jedoch unter Verwendung der Verbindung des Beispiels 46, wird die Titelverbindung als Schaum mit den untenstehende Kenndaten erhalten.

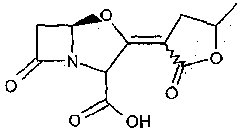

**PatAZ1814k mehr polare I-Reihen ¹H-NMR** (DMSO-d₆, 400 MHz): δ 5,94 (d, J =2,7 Hz, 1 H); 5,40 (s, 1 H); 4,73-4,66 (m, 1 H); 3,74 (dd, J = 3,0, 17,1 Hz, 1 H); 3,27 (d überlappend mit H₂O); 3,07 (ddd, J = 1,2, 7,8, 16,1 Hz, 1 H); 2,49 (m vergraben unter DMSO); 1,32 (d, J = 6,2 Hz, 3 H).

### Beispiel 51

### (5R)-3-(1-Carboxybutyliden)-7-oxo-4-oxa-1-aza-bicyclo[3.2.0]heptan-2-carbonsäure

Analog zu dem im Beispiel 49 beschriebenen Verfahren, jedoch unter Verwendung der Verbindung des Beispiels 24, wird die Titelverbindung als Schaum mit den untenstehende Kenndaten erhalten.

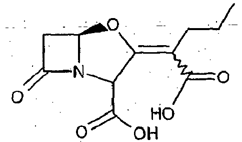

**PätAZ8285i ¹H-NMR** (DMSO-d₆, 400 MHz): δ 13,5-12,2 (br. s, 1 H); 5,81 (d, J = 2,7 Hz, 1 H); 5,38 (s, 1 H); 3,69 (dd, J = 2,9, 17,0 Hz, 1 H); 3,19 (d, J = 17,0 Hz, 1 H); 2,23 (t, J = 7,5 Hz, 2 H); 1,45-1,35 (m, 2 H); 0,85 (t, J = 7,4 Hz, 3 H).

### Beispiel 52

### (5R)-3-(1-Carboxy-4-phenylbutyliden)-7-oxo-4-oxa-1-aza-bicyclo[3.2.0]heptan-2-carboxylat1,1,3,3-tetramethylbutylammonium

Analog zu dem im Beispiel 49 beschriebenen Verfahren, jedoch unter Verwendung der Verbindung des Beispiels 26, wird (5R)-3-(1-Carboxy-4-phenylbutyliden)-7-oxo-4-oxa-1-aza-bicyclo[3.2.0]heptan-2-carbonsäure als Schaum erhalten. Eine Mischung aus dem Schaum, Ethylacetat und 1,1,3,3-Tetramethylbutylamin wird bei 0-5°C gerührt. Nach 30 Minuten wird das Lösungsmittel unter Vakuum entfernt und der Rückstand in 1 ml Dichlormethan aufgelöst, 3 ml Petrolether werden in kleinen Portionen hinzugefügt, und dies wird 15 Minuten lang stehen gelassen. Ein Präzipitat, das sich gebildet hat, wird filtriert, mit Petrolether gewaschen und getrocknet. Die Titelverbindung wird als Pulver mit den untenstehenden Kenndaten erhalten.

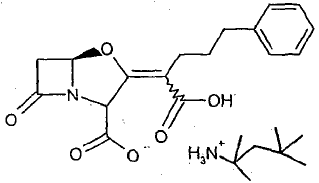

**PatAZ8230i ¹H-NMR** (DMSO-d₆, 400 MHz): δ 7,27-7,12 (m, 5 H); 5,67 (d, J = 2,4 H, 1 H); 5,19 (s, 1 H); 3,61 (dd, J = 17,0, 2,8 Hz, 1 H); 2,99 (d, J = 17,0 Hz, 1 H); 1,61 (m, 2 H); 1,44 (s, 2 H); 1,17 (s, 6 H); 0,98 (s, 9 H).

### Beispiel 53

### (5R)-3-(1-Carboxy-4-phenylbutyliden)-7-oxo-4-oxa-1-aza-bicyclo[3.2.0]heptan-2-carbonsäuremethylester

Analog zu dem im Beispiel 49 beschriebenen Verfahren, jedoch unter Verwendung der Verbindung des Beispiels 34, wird die Titelverbindung als Öl mit den untenstehende Kenndaten erhalten.

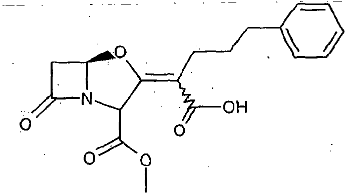

**Pat AZ8207i ¹H-NMR:** (DMSO-d₆, 400 MHz): δ 12,42 (br. s, 1 H); 7,28 -7,25 (m, 2 H); 7,19-7,14 (m, 3 H); 5,85 (d, J = 2,8 Hz, 1 H); 5,45 (s, 1 H); 3,69 (dd, J = 3,0, 17,2 Hz, 1 H); 3,66 (s, 3 H); 3,26 (d, J = 17,0 Hz, 1 H); 2,61-2,53 (m, 2 H); 2,32 (t, J = 7,3 Hz, 2 H); 1,75-1,65 (m, 2 H).

### Beispiel 54

### (5R)-3-(1-Carboxybutyliden)-7-oxo-4-oxa-1-aza-bicyclo[3.2.0]heptan-2-carbonsäuremethylester

Analog zu dem im Beispiel 49 beschriebenen Verfahren, jedoch unter Verwendung der Verbindung des Beispiels 32, wird die Titelverbindung als Öl mit den untenstehenden Kenndaten erhalten.

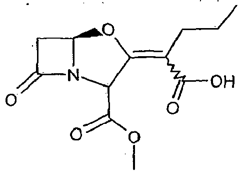

**PatAZ8300i ¹H-NMR** (DMSO-d₆, 400 MHz): 12,49 (br. s); 5,84 (d, J = 2,4 Hz, 1 H); 5,45 (s, 1 H); 3,70 (dd, J = 3,0, 17,1 Hz, 1 H); 3,66 (s, 3 H); 3,25 (d, J = 17,2 Hz, 1 H); 2,25 (t, J = 7,4 Hz, 2 H); 1,48-1,37 (m, 2 H); 0,86 (t, J = 7,4 Hz, 3 H).

### Beispiel 55

### (5R)-3-(1-Hydroxymethylbutyliden)-7-oxo-4-oxa-1-aza-bicyclo[3.2.0]heptan-2-carboxylat1,1,3,3-tetramethylbutylammonium

Analog zu dem im Beispiel 49 beschriebenen Verfahren, jedoch unter Verwendung der Verbindung des Beispiels 38, wird (5R)-3-(1-Hydroxymethylbutyliden)-7-oxo-4-oxa-1-aza-bicyclo[3.2.0]heptan-2-carbonsäure als Öl erhalten. Eine Mischung aus dem Öl, Ethylacetat und 1,1,3,3-Tetramethylbutylamin wird bei Raumtemperatur 10 Minuten lang gerührt. Die Lösungsmittel werden unter Vakuum entfernt. Die Titelverbindung wird als Öl mit den untenstehenden Kenndaten erhalten.

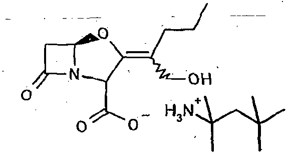

**PatAZ2537i ¹H-NMR** (DMSO-d₆, 400 MHz): Mischung von Isomeren; Daten des Hauptisomers: δ 5,48 (d, J = 2,7 Hz, 1 H); 4,89 (s, 1 H); 3,94 & 3,81 (zwei Dubletten als ABq, J = 13,0 Hz, 2 H); 3,44 (dd, J = 2,7, 16,3 Hz, 1 H); 2,77 (d, J = 16,5 Hz, 1 H); 2,05-1,93 (m, 2 H); 1,39-1,33 (überlappende Multipletten); 0,82 (t, J = 7,4 Hz, 3 H); 1,35 (s, 2 H); 1,29 (s, 6 H); 0,98 (s, 9 H).

### Beispiel 56

### (5R)-3-[2-Hydroxyethyliden]-7-oxo-2-propyl-4-oxa-1-aza-bicyclo[3.2.0]heptan-2-carboxylat1,1,3,3 -tetramethylbutylammonium

Analog zu dem im Beispiel 49 beschriebenen Verfahren, jedoch unter Verwendung der Verbindung des Beispiels 40, wird (5R)-3-[2-Hydroxy-eth-(Z)-yliden]-7-oxo-2-propyl-4-oxa-1-aza-bicyclo[3.2.0]heptan-2-carbonsäure als Öl erhalten. Eine Mischung aus dem Öl, Ethylacetat und 1,1,3,3-Tetramethylbutylamin wird bei Raumtemperatur gerührt. Ein Präzipitat, das sich gebildet hat, wird filtriert und getrocknet. Die Titelverbindung wird als Pulver mit den untenstehenden Kenndaten erhalten.

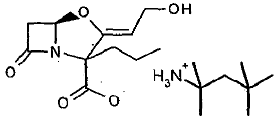

**PatAZ2536i ¹H-NMR** (D₂O, 400 MHz): Mischung von Isomeren; Hauptisomer: δ 5,59 (d, J = 2,4 H, 1 H); 4,87 (t, J = 7,4 Hz, 1 H); 4,23 (dd, J = 7,8, 12,2 Hz, 1 H); 4,16 (dd, J = 7.1, 12,1 Hz, 1 H); 3,47 (dd, J= 2,9, 16,8 Hz, 1 H); 3,07 (d, J = 16,8 H, 1 H); 2,16 (ddd, J = 13,8, 12,2, 4,5 Hz, 1 H); 1,90 (ddd, J = 14,1, 12,2, 4,0 Hz, 1 H); 1,78-1,67 (überlappende Multipletten); 1,43-1,30 (überlappende Multipletten); 0,97 (t, J = 7,3 Hz, 3 H); 1,70 (s, 2 H); 1,47 (s, 6 H); 1,06 (s, 9 H).

### Beispiel 57

### (5R)-3-(1-Methoxycarbonyl-4-phenylbutyliden)-7-oxo-4-oxa-1-aza-bicyclo[3.2.0]heptan-2-carbonsäuremethylester

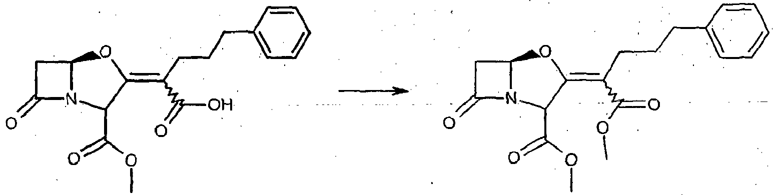

Eine Mischung aus der Verbindung des Beispiels 53 (22 mg), 1 ml Ethylacetat und 0,2 ml einer etherischen Lösung von Diazomethan wird bei 0-5°C 25 Minuten lang gerührt. Die Lösungsmittel werden unter Vakuum entfernt, um einen Rückstand zu erhalten. Der Rückstand wird an einer kurzen SiO₂-Säule chromatographiert. Die Titelverbindung wird als Öl mit den untenstehenden Kenndaten erhalten.

**PatAZ8208i ¹H-NMR** (DMSO-d₆, 400 MHz): 7,28 -7,25 (m, 2 H); 7,19 -7,16 (m, 3 H); 5,89 (d, J = 2,4 Hz, 1 H); 5,47 (s, 1 H); 3,71 (dd, J = 3,1, 17,0 Hz, 1 H); 3,69 (s, 3 H); 3,61 (s, 3 H); 3,28 (d, J = 17,0 Hz, 1 H); 2,61-2,52 (m, 2 H); 2,34 (t, J = 7,5 Hz, 2 H); 1,77-1,64 (m, 2 H).

### Beispiel 58

### (5R)-3-[5-(Benzylbenzyloxycarbonylamino)-1-carboxypent-3-enyliden]-7-oxo-4-oxa-1-aza-bicyclo[3.2.0]heptan-2-carbonsäurebenzylester

Analog zu dem in den Beispielen 24, 25 beschriebenen Verfahren, jedoch unter Verwendung der Verbindung des Beispiels 21, wird die Titelverbindung als Öl mit den untenstehende Kenndaten erhalten.

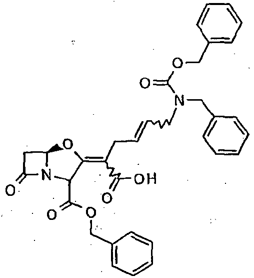

**PatAZ 7229k ¹H-NMR** (DMSO-d₆ 400 MHz): δ 12,53 (br s, 1 H); 7,39-7,16 (m, 15 H); 5,85 (d, J = 2,7 Hz, 1 H); 5,55 (s, 1 H); 5,55-5,45 (br, 1 H); 5,42-5,35 (m, 1 H); 5,14 (s, 2 H); 5,09 (s, 2 H); 4,34 (s, 2 H); 3,72 (dd, J = 2,9, 17,0 Hz, 1 H); 3,75-3,70 (br, 2 H); 3,21 (d, J = 17,0 Hz, 1 H); 2,98 (d, J = 5,5 Hz, 2 H).

### Beispiel 59

### (5R)-3-[5-Iodomethyldihydrofuran-3-yliden]-7-oxo-4-oxa-1-aza-bicyclo[3.2.0]heptan-2-carbonsäurebenzylester

Analog zu dem in den Beispielen 41, 42 beschriebenen Verfahren, jedoch unter Verwendung der Verbindung des Beispiels 38, wird die Titelverbindung als Öl mit den untenstehenden Kenndaten erhalten.

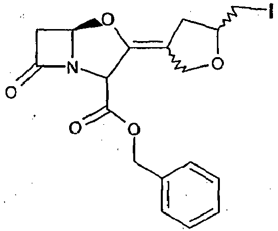

**PatAZ72281c ¹H-NMR** (DMSO-d₆, 500 MHz): Mischung von zwei Isomeren; δ 7,41-7,34 (m, 4,5 H); 5,73 (2 überlappende Dubletten, J = 2,8, 3,2 Hz, 0,9 H); 5,45 (s, 0,3 H); 5,39 (s, 0,6 H); 5,21 & 5,17 (2 Dubletten als ABq, J = 12,4 Hz, 1,2 H); 5,18 (s, 0,6 H); 4,48 (d, J = 12,4 Hz, 0,3 H); 4,33 (d, J = 12,4 Hz, 0,6 H); 4,19 (d, J = 12,4 Hz, 0,6 H); 4,01-3,96 (überlappende Multipletten, 0,9 H); 3,89-3,84 (Quintett, J ca. 6 Hz, 0,3 H); 3,62 (dd, J = 3,2, 16,9 Hz, 0,9 H); 3,38 (dd, J = 5,3, 10,3 Hz, 0,3 H); 3,30 (m); 3,25 (dd, J = 10,2, 5,8 Hz, 0,6 H); 3,11 (d, J = 17,0 Hz, 0,6 H); 3,10 (d, J = 17,0 Hz, 0,3 H); 2,75 (dd, J = 6,8, 16,1 Hz, 0,6 H); 2,64 (ddd, J = 1,4, 6,8, 16,2 Hz, 0,3 H); 2,28-2,21 (2 überlappende dd, J ca. 7,3, 16,1 Hz, 0,9 H).

Die hemmende Wirkung der erfindungsgemäßen Verbindungen auf β-Lactamasen wird in den folgenden Beispielen gezeigt:

### Inhibitionsstudie hinsichtlich zeltfreier β-Lactamase

Die hemmenden Konzentrationen (IC50, [µM]) der β-Lactamase-Hemmer gegen gereinigte TEM-1-, SHV-1- und AmpC-ß-Lactamasen werden bewertet, indem die Konzentration des Hemmstoffs bestimmt wird, bei der 50% der Nitrocefin-Hydrolyse durch das spezielle Enzym gehemmt wird. Analysen werden mit ß-Lactamasen durchgeführt, die im pET-System (Novagen, San Diego, CA) ohne Signalpeptide exprimiert wurden. Sie enthalten eine N-terminale hexa-Histidin-Markierung, die zur Reinigung an Ni-NTA (Qiagen, Hilden, Deutschland) verwendet wird. Die Verbindungen werden als 50 mM-Ausgangsmaterialien in DMSO hergestellt und im Puffer P1 (50 mM Phosphat, pH 7) zu einer endgültigen Konzentration von 10%igem DMSO verdünnt. Alle weiteren Verdünnungen erfolgen in P2 (P1 mit 10%igem DMSO). Die Enzym- und Verbindungsverdünnungen werden bei 37°C 10 Minuten lang vorinkubiert und die Reaktion wird mit dem Hinzufügen von vorgewärmtem (37°C) Nitrocefin bei einer endgültigen Konzentration von 50 mM gestartet. Die Veränderung der Absorption bei 490 nm wird bei 37°C in einem SPECTRAMAX 384 PLUS-Miktroplattenleser (Molecular Devices, Sunnyvale, CA) unter Verwendung von 96-Mulden-Platten 10 Minuten lang mit 30 Sekunden-Intervallen verfolgt. Die Anfangsgeschwindigkeit wird bestimmt, und IC50-Werte werden durch eine nichtlineare Regression und eine sigmoidale Dosis-Wirkung-Analyse mit der PRISM 4.0-Software (Graphpad Software Inc., San Diego, CA) berechnet. IC₅₀-Daten werden in µM mit einem Vertrauensbereich von 95% ausgedrückt und aus zumindest zwei unabhängigen Versuchen errechnet.

### IC₅₀-Werte der Verbindungen der vorliegenden Erfindung und der kommerziell erhältlichen Verbindungen hinsichtlich ß-Lactamase-Enzymen:

| Verbindung | Enzym | IC₅₀ [µM] |
|---|---|---|
| Clavulansäure Kalium Salz | AmpC | 137.6647 |
| Clavulansäure Kalium Salz | SHV-1 | 0.0303 |
| Clavulansäure Kalium Salz | TEM-1 | 0.0295 |
| Sulbactam Natrium Salz | AmpC | 25.4954 |
| Sulbactam Natrium Salz | SHV-1 | 3.9150 |
| Sulbactam Natrium Salz | TEM-1 | 1.0659 |
| Tazobactam Natrium Salz | AmpC | 1.8082 |
| Tazobactam Natrium Salz | SHV-1 | 0.2220 |
| Tazobactam Natrium Salz | TEM-1 | 0.0172 |
| Beispiel 1 | AmpC | 10.4476 |
| Beispiel 1 | TEM-1 | 0.0666 |
| Beispiel 2 | AmpC | 58.7670 |
| Beispiel 2 | TEM-1 | 0.9454 |
| Beispiel 3 | AmpC | 95.0730 |
| Beispiel 3 | TEM-1 | 8.3863 |
| Beispiel 4 | AmpC | 2.4695 |
| Beispiel 4 | TEM-1 | 0.0890 |
| Beispiel 5 | AmpC | 5.8200 |
| Beispiel 5 | SHV-1 | 7.1435 |
| Beispiel 5 | TEM-1 | 4.9233 |
| Beispiel 6 | AmpC | 17.0795 |
| Beispiel 6 | SHV-1 | 3.9503 |
| Beispiel 6 | TEM-1 | 2.7923 |
| Beispiel 7 | AmpC | 5.3763 |
| Beispiel 7 | SHV-1 | 0.2169 |
| Beispiel 7 | TEM-1 | 0.0784 |
| Beispiel 8 | AmpC | 115.3075 |
| Beispiel 8 | TEM-1 | 5.6422 |
| Beispiel 9 | AmpC | 14.2710 |
| Beispiel 9 | SHV-1 | 1.0430 |
| Beispiel 9 | TEM-1 | 0.8275 |
| Beispiel 10 | AmpC | 16.0350 |
| Beispiel 10 | TEM-1 | 0.1399 |
| Beispiel 11 | AmpC | 532.0000 |
| Beispiel 11 | TEM-1 | 7.3067 |
| Beispiel 12 | AmpC | 29.3960 |
| Beispiel 12 | TEM-1 | 5.8977 |
| Beispiel 13 | AmpC | 0.9943 |
| Beispiel 13 | SHV-1 | 0.0477 |
| Beispiel 13 | TEM-1 | 0.6394 |
| Beispiel 14 | AmpC | 136.5686 |
| Beispiel 14 | SHV-1 | 85.1150 |
| Beispiel 14 | TEM-1 | 19.6320 |
| Beispiel 15 | AmpC | 55.9574 |
| Beispiel 15 | SHV-1 | 4.4272 |
| Beispiel 15 | TEM-1 | 4.2683 |
| Beispiel 16 | AmpC | 0.9414 |
| Beispiel 16 | SHV-1 | 0.0805 |
| Beispiel 16 | TEM-1 | 0.0652 |
| Beispiel 17 | AmpC | 36.0330 |
| Beispiel 17 | SHV-1 | 21.3035 |
| Beispiel 17 | TEM-1 | 13.2596 |
| Beispiel 18 | AmpC | 18.9543 |
| Beispiel 18 | SHV-1 | 0.0139 |
| Beispiel 18 | TEM-1 | 0.0271 |
| Beispiel 19 | AmpC | 79.0345 |
| Beispiel 19 | SHV-1 | 0.6166 |
| Beispiel 19 | TEM-1 | 0.8706 |
| Beispiel 24 | AmpC | 4.9780 |
| Beispiel 24 | SHV-1 | 0.0622 |
| Beispiel 24 | TEM-1 | 0.0306 |
| Beispiel 26 | AmpC | 2.2006 |
| Beispiel 26 | SHV-1 | 0.0363 |
| Beispiel 26 | TEM-1 | 0.0092 |
| Beispiel 27 | AmpC | 6.0248 |
| Beispiel 27 | SHV-1 | 0.6243 |
| Beispiel 27 | TEM-1 | 0.4261 |
| Beispiel 28 | AmpC | 11.7498 |
| Beispiel 28 | SHV-1 | 9.3179 |
| Beispiel 28 | TEM-1 | 45.6844 |
| Beispiel 32 | AmpC | 2.8648 |
| Beispiel 32 | SHV-1 | 0.3390 |
| Beispiel 32 | TEM-1 | 0.1215 |
| Beispiel 34 | AmpC | 0.0884 |
| Beispiel 34 | SHV-1 | 0.0503 |
| Beispiel 34 | TEM-1 | 0.0217 |
| Beispiel 35 | AmpC | 41.0621 |
| Beispiel 35 | SHV-1 | 2.3072 |
| Beispiel 35 | TEM-1 | 1.5615 |
| Beispiel 36 | AmpC | 4.1861 |
| Beispiel 36 | SHV-1 | 0.6423 |
| Beispiel 36 | TEM-1 | 0.4076 |
| Beispiel 37 | AmpC | 8.2748 |
| Beispiel 37 | SHV-1 | 0.1413 |
| Beispiel 37 | TEM-1 | 0.0442 |
| Beispiel 38 | AmpC | 2.7249 |
| Beispiel 38 | SHV-1 | 6.8103 |
| Beispiel 38 | TEM-1 | 8.0160 |
| Beispiel 38a | AmpC | 32.1830 |
| Beispiel 38a | SHV-1 | 1.7131 |
| Beispiel 38a | TEM-1 | 1.9486 |
| Beispiel 39 | AmpC | 4.1188 |
| Beispiel 39 | SHV-1 | 5.6099 |
| Beispiel 39 | TEM-1 | 4.1249 |
| Beispiel 40 | AmpC | 85.9540 |
| Beispiel 40 | TEM-1 | 23.6215 |
| Beispiel 41 | AmpC | 0.2854 |
| Beispiel 41 | SHV-1 | 0.0004 |
| Beispiel 41 | TEM-1 | 0.0002 |
| Beispiel 42 | AmpC | 0.3848 |
| Beispiel 42 | SHV-1 | 0.0008 |
| Beispiel 42 | TEM-1 | 0.0017 |
| Mixture of Beispiel 43 and Beispiel 44 | AmpC | 0.1260 |
| Mixture of Beispiel 43 and Beispiel 44 | SHV-1 | 0.0043 |
| Mixture of Beispiel 43 and Beispiel 44 | TEM-1 | 0.0035 |
| Beispiel 45 | AmpC | 1.0697 |
| Beispiel 45 | SHV-1 | 0.0034 |
| Beispiel 45 | TEM-1 | 0.0017 |
| Beispiel 46 | AmpC | 0.3006 |
| Beispiel 46 | SHV-1 | 0.0005 |
| Beispiel 46 | TEM-1 | 0.0004 |
| Beispiel 48 | AmpC | 0.3046 |
| Beispiel 48 | SHV-1 | 0.0042 |
| Beispiel 48 | TEM-1 | 0.0024 |
| Beispiel 49 | AmpC | 6.0817 |
| Beispiel 49 | SHV-1 | 0.0026 |
| Beispiel 49 | TEM-1 | 0.0018 |
| Beispiel 50 | AmpC | 26.9820 |
| Beispiel 50 | SHV-1 | 0.0032 |
| Beispiel 50 | TEM-1 | 0.0042 |
| Beispiel 51 | AmpC | 414.6187 |
| Beispiel 51 | SHV-1 | 0.4357 |
| Beispiel 51 | TEM-1 | 0.2540 |
| Beispiel 53 | AmpC | 0.5415 |
| Beispiel 53 | SHV-1 | 0.2190 |
| Beispiel 53 | TEM-1 | 0.0728 |
| Beispiel 54 | AmpC | 3.4215 |
| Beispiel 54 | SHV-1 | 0.2946 |
| Beispiel 54 | TEM-1 | 0.0970 |
| Beispiel 55 | TEM-1 | 118.4030 |
| Beispiel 56 | AmpC | 57.4200 |
| Beispiel 56 | TEM-1 | 17.7890 |
| Beispiel 57 | AmpC | 5.3274 |
| Beispiel 57 | SHV-1 | 0.0226 |
| Beispiel 57 | TEM-1 | 0.0106 |
| Beispiel 58 | AmpC | 4.5135 |
| Beispiel 58 | SHV-1 | 0.0006 |
| Beispiel 58 | TEM-1 | 0.0014 |
| Beispiel 59 | AmpC | 6.4502 |
| Beispiel 59 | SHV-1 | 3.6044 |
| Beispiel 59 | TEM-1 | 4.3923 |

Die antibakterielle Wirksamkeit der erfindungsgemäßen Verbindungen in Kombination mit Antibiotika wird in den folgenden Beispielen gezeigt:

### in vitro-Synergieversuche

### Materialien und Verfahren

### Antibakterielle Wirkstoffe:

Stammlösungen der Testverbindungen und Kontrollantibiotika werden gemäß den NCCLS-Richtlinien (NCCLS (National Commitee for Clinical Laboratory Standards) 12(3). 2000. M7-A4) in destilliertem Wasser hergestellt. Lipophile Verbindungen mit geringer Wasserlöslichkeit werden zunächst in DMSO (Dimethylsulfoxid, 20 %) aufgelöst und mit Wasser oder Mueller-Hinton-Bouillon (MHB) weiter verdünnt. Alle Arzneimittelgewichte werden in Hinblick auf die Salzformen korrigiert und beziehen sich auf die reine Arzneimittelsubstanz.

### Bakterienstämme:

Alle untersuchten Stämme und klinischen Isolate werden entweder von der American Type Culture Collection (ATCC) erworben, von A. Georgopoulos (Wiener Allgemeines Krankenhaus, Wien, Österreich) und I. Chopra (Leeds, UK) freundlicherweise zur Verfügung gestellt oder von der SENTRY-Studie (F.J. Schmitz, Minden, Deutschland) oder der Sandoz-Kultursammlung erhalten.

Alle Stammidentitäten werden durch BBL CrystalTM-Identifikationssysteme (Beckton Dickinson, Cockeysville, Maryland, USA) bestätigt. Stammkulturen werden aus Nährlösungen hergestellt, die bei 35°C ohne Bewegung und anschließendes Hinzufügen von 5 %igem DMSO (v/v, endgültige Konzentration) als Kryoschutzmittel 18-22 Stunden lang gezüchtet wurden, und in flüssigem Stickstoff gefroren gelagert.

Das Vorhandensein von ß-Lactamase-Genen wird mittels PCR bewiesen, wobei Primer verwendet werden, die für den jeweiligen ß-Lactamasetyp spezifisch sind.

### Test Methode

Die als MIC ausgedrückte bakterielle Anfälligkeit wird durch eine Mikronährlösungs-Verdünnungstechnik und eine Agar-Verdünnungstechnik bestimmt, wie von den anerkannten Standard-Referenzempfehlungen des NCCLS empfohlen. Die ausgewählten Antibiotika werden durch eine serielle zweifache Verdünnung, die entweder von 256 µg/ml bis 0,125 µg/ml oder von 25,6 bis 0,0125 µg/ml reicht, verdünnt. Bei Kombination mit einem ß-Lactamase-Hemmer wird der Hemmstoff bei einer konstanten Konzentration von 17 oder 68 µM hinzugefügt.

Das Inokulum wird, wie vom NCCLS beschrieben, durch das direkte Kolonie-suspensionsverfahren hergestellt. Zu diesem Zweck werden die Bakterien bei 35°C 24 Stunden lang auf Blutagarplatten gezüchtet. Anschließend werden 3-5 einzelne Kolonien in 5 ml flüssigem Medium suspendiert. Die Bakterien werden danach 5 Stunden lang inkubiert, um die logarithmische Wachstumsphase zu erreichen, und anschließend mit Medium oder NaCl (0,86 %) verdünnt, um eine endgültige Zahl von lebenden Zellen, die 10⁸ CFU/ml entspricht, zu erhalten. Daraufhin wird die bakterielle Suspension 1:10 verdünnt und 1 µl (10⁴ CFU; endgültiges Inokulum 10⁵ CFU/ml) wird durch einen Multipoint-Inokulator (Dynatech, Chantilly, Virginia, USA) auf das Testmedium, welches die antibakteriellen Substanzen enthält, übertragen. Die Platten werden dann bei 35°C 24 Stunden lang inkubiert. Die MIC wird optisch bestimmt und als die minimale Hemmkonzentration [µg/ml] eines Antibiotikums, bei der kein sichtbares Bakterienwachstum stattfindet, definiert.

### Minimale Hemmstoffkonzentrationen (MIC) von Amoxicillin, Ceftazidim und Cefepim allein und in Gegenwart der Verbindung des Beispiels 49 in der festgelegten Konzentration:

| | | Amoxicillin MIC, µg/ml | | Ceftazidim MIC, µg /ml | | Cefepim MIC, µg /ml | |
|---|---|---|---|---|---|---|---|
| | ATCC Nr. | allein | + 68 µM Beispiel 49 | allein | + 68 µM Beispiel 49 | allein | +17 µM Beispiel 49 |
| K. pneumoniae | 700603 | > 25,6 | 3,2 | > 25,6 | 0,2 | 0,8 | 0,2 |
| E. cloacae | 83989 | nicht untersucht | nicht untersucht | > 25,6 | 12,8 | 0,8 | 0,8 |
| C. freundii | 7023771 | nicht untersucht | nicht untersucht | > 25,6 | 3,2 | 0,8 | 0,4 |

### Pharmakokinetik

Die erfindungsgemäßen Verbindungen zeigen nach subkutaner oder p.o.-Verabreichung eine biologische Verfügbarkeit in Tieren, wie in den folgenden Beispielen gezeigt wird.

Die Verbindung des Beispiels 49 führte nach einer oralen Anwendung von 25 mg/kg (formuliert in 650 mg Cremophor EL + 1 ml 96%igem Ethanol + 4,575 ml 5%iger Glucose in Salzlösung) bei Mäusen nach 15 Minuten zu einer Plasma-Höchstkonzentration (Cₘₐₓ) von 2,22 µg /ml, der AUCₜₒₜ erreichte 65,123 µg/ml*min, und die T_{half} betrug 18,90 Minuten.

Die subkutane Anwendung von 10 mg/kg (in 0,05 M NaPO₄-Puffer, pH 7,2) bei Mäusen führte nach 5. Minuten zu einer Cₘₐₓ von 5,6 µg/ml, der AUCₜₒₜ betrug 125,65 µg/ml*min, und eine T_{half} von 12,27 Minuten wurde erreicht.

Die Werte der PK-Parameter wurden durch eine nicht kompartimentale Standardanalyse unter Verwendung von Kinetica 4.2 (InnaPhase) errechnet. Die Plasmakonzentrationswerte unterhalb der Quantifizierungsgrenze wurden als 0 genommen. Die maximale Plasma-konzentration (Cₘₐₓ) und die Zeit zum Erreichen von Cₘₐₓ wurden aus den beobachteten Daten ermittelt.

### Antibakterielle in vivo-Aktivität

Die erfindungsgemäßen Verbindungen zeigen bei gleichzeitiger Verabreichung eines Antibiotikums eine antibakterielle Wirksamkeit in Tieren, wie in den nachfolgenden Beispielen gezeigt wird.

Ein Sepsis-Modell in weiblichen NMRI-Mäusen wurde angewandt, wobei TEM-1-produzierendes *E. coli*-B269 (klinisches Isolat) als infizierender Bakterienstamm eingesetzt wurde. Im Allgemeinen stellte das endgültige Inokulum, das mit 0,3 ml pro Maus intraperitoneal verabreicht wurde, bei systemischen Infektionen innerhalb von 48 Stunden eine 100% letale Konzentration dar.

Eine Kombination des β-Lactam-Antibiotikums Cefepim mit der Verbindung des Beispiels 49 wurde mit Cefepim allein verglichen. Der subkutane Behandlungsplan für das Antibiotikum in verschiedenen Dosierungen erfolgte gleichzeitig und 3 Stunden nach der Infektion. Die Testverbindung (Verbindung des Beispiels 49) wurde in einer fixen Dosis von 20 mg/kg über denselben Weg verabreicht, und zwar gleichzeitig bzw. 1,5, 3 und 4,5 Stunden nach der Infektion.

Nach einer Behandlung mit der Kombination von Cefepim und der Testverbindung führte eine Dosis von z.B. 0,5 mg/kg Cefepim zum Überleben aller Tiere in dieser Behandlungsgruppe, während nach einer Behandlung mit vergleichbaren Dosierungen des Antibiotikums allein kein Tier überlebte.

## Patentansprüche

1. Eine Verbindung der Formel I 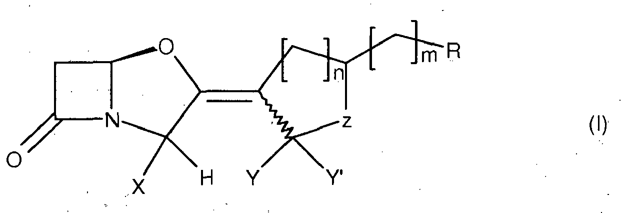 wobei
X COOH oder ein Salz davon,
COOR₁, wobei R₁ optional substitutiertes C₁₋₄ Alkyl darstellt,
CONR₂R₃, wobei R₂ und R₃ unabhängig voneinander H oder C₁₋₅ Alkyl, das optional ein oder mehrere Heteroatome aus O, S und N enthält und/oder optional substituiert ist, oder
CON(R₂)-CH(R₄)-R₅, wobei
R₂ wie oben definiert ist,
R₄ eine Gruppe ist, die gewönlich in der α Stelle von α-Aminosäuren vorkommt, und
R₅ COOH oder ein Salz davon, COOR₁, wobei R₁ wie oben definiert ist; oder CONR₂R₃ ist, wobei R₂ and R₃ wie oben definiert sind,
darstellt,
Y and Y' beide H sind oder zusammen =O darstellen,
Z O, NR₂ oder N-CH(R₄)-R₅, wobei R₂, R₄ und R₅ wie oben definiert sind, ist, n 1 oder 2 ist,
m eine Zahl von 0 bis 4 ist und
R H, oder
eine gesättigte oder ungesättigte Kette mit 1 bis 8 Kohlenstoffatomen, die optional durch einen 5 oder 6-gliediigen, nichtaromatischen Ring, der ein oder mehrere Heteroatome, ausgewählt aus O, S und N, enthält, oder durch Aryl, Alkyl oder Cycloalkyl substituiert ist, optional eine oder mehrere Heteroatome ausgewählt aus O, S und N enthält, und/oder ein- oder mehrfach substituiert ist durch Heteroatome ausgewählt aus O, S und N, Halogene Cl, Br, I und F und/oder Azido N₃, ist.

2. Verbindung nach Anspruch 1 in einer pharmazeutisch akzeptablen Salzform, vorzugsweise in Form eines Kalium Salzes.

3. Verbindung nach Anspruch 1 oder 2, wobei R₁ Methyl, vorzugsweise substituiert mit einer Pivaloyloxygruppe, darstellt.

4. Verbindung nach einem der Ansprüche 1 bis 3, wobei R₂ und R₃ unabhängig voneinander substituiertes C₁₋₄ Alkyl oder C₅₋₆ Cycloalkyl darstellen.

5. Verbindung nach einem der Ansprüche 1 bis 4, wobei R₄ Methyl oder Benzyl darstellt.

6. Verbindung nach einem der Ansprüche 1 bis 5, wobei R eine gesättigte oder ungesättigte Kette mit 1 bis 8 Kohlenstoffatomen, die optional durch einen 5 oder 6-gliedrigen nichtaromatischen Ring substituiert ist, der ein oder mehrere Heteroatome, ausgewählt aus O, S und N, enthält, oder durch Aryl, Alkyl oder Cycloalkyl substituiert ist, darstellt.

7. Verbindung nach einem der Ansprüche 1 bis 6, wobei R eine gesättigte oder ungesättigte Kette mit 1 bis 8 Kohlenstoffatomen, substituiert durch ein oder mehrere Heteroatome ausgewählt aus O, S und N, wobei die O und S Herteroatome als Ester, Carbonate oder Äther, und die N Heteroatome alkyliert oder als Amide oder Carbamate geschützt sind, darstellt.

8. Verbindung nach Anspruch 1 mit der Formel III

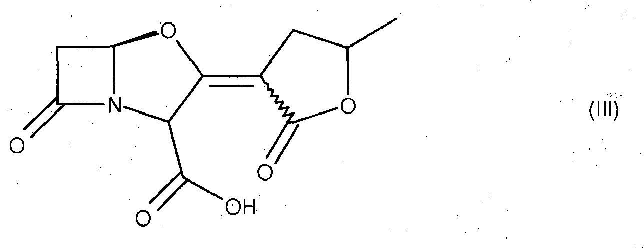

9. Zwischenverbindung der Formel IV.

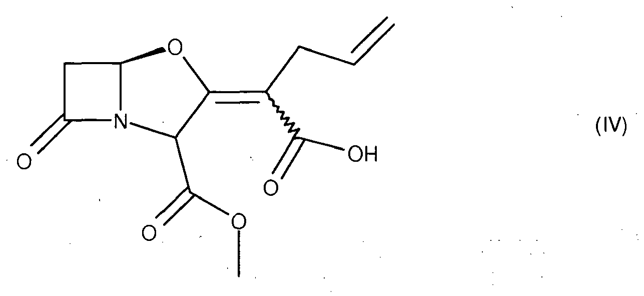

10. Zwischenverbindung der Formel V

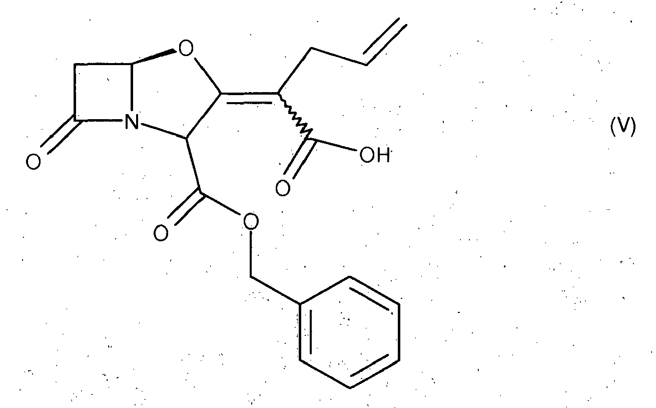

11. Verbindung nach einem der Ansprüche 1 bis 8 zur Anwendung als ein Medikament.

12. Verbindung nach einem der Ansprüche 9 oder 10 zur Anwendung als ein Medikament.

13. Pharmazeutische Zusammensetzung enthaltend eine Verbindung einer der Ansprüche 1 bis 8, und optional zusätzlich ein β-Lactam Antibiotikum aus der Klasse der Penicilline oder Cephalosporine.

14. Pharmazeutische Zusammensetzung enthaltend eine Verbindung gemäß einem der Ansprüche 9 oder 10, und optional zusätzlich ein β-Lactam Antibiotikum aus der Klasse der Penicilline oder Cephalosporine.

## Claims

1. A compound of formula I 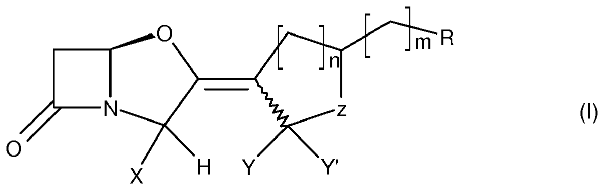 wherein
X denotes
COOH or a salt thereof,
COOR₁, wherein R₁ is optionally substituted C₁₋₄ alkyl,
CONR₂R₃, wherein R₂ and R₃ independently of each other are H or C₁₋₅ alkyl, optionally comprising one or more heteroatoms from O, S and N, and/or optionally substituted, or
CON(R₂)-CH(R₄)-R₅, wherein
R₂ is as defined above,
R₄ is a group normally occurring in the α position of α-amino acids,and R₅ is COOH or a salt thereof, COOR₁, wherein R₁ is as defined above; or CONR₂R₃
wherein R₂ and R₃ are as defined above,
Y and Y' both are H, or together denote =O,
Z is O, NR₂ or N-CH(R₄)-R₅, wherein R₂, R₄ and R₅ are as defined above,
n is 1 or 2,
m is a number from 0 to 4, and
R is H, or
a saturated or unsaturated chain having 1 to 8 carbon atoms, optionally substituted
by a 5- or 6 membered non-aromatic ring, having one or more heteroatoms selected from O, S and N, or
by aryl, alkyl or cycloalkyl,
optionally comprising one or more heteroatoms selected from O, S and N,
and/or one or morefold substituted by heteroatoms selected from O, S and N, halogen Cl, Br, I and F and/or azido N₃.

2. Compound according to claim 1 in a pharmaceutically acceptable salt form, in particular in the form of a potassium salt.

3. Compound according to claim 1 or 2, wherein R₁ is methyl, in particular substituted with a pivaloyloxy group.

4. Compound according to any one of claims 1 to 3, wherein R₂ and R₃ independently of each other are substituted C₁₋₄ alkyl or C₅₋₆ cycloalkyl.

5. Compound according to any one of claims 1 to 4, wherein R₄ is methyl or benzyl.

6. Compound according to any one of claims 1 to 5, wherein R is a saturated or unsaturated chain having 1 to 8 carbon atoms,
optionally substituted by a 5 oder 6-membered non aromatic ring comprising one or more heteroatoms selected from O, S and N,
or substituted by aryl, alkyl or cycloalkyl.

7. Compound according to any one of claims 1 to 6, wherein R is a saturated or unsaturated chain having 1 to 8 carbon atoms, substituted by one or more heteroatoms, selected from O, S and N, wherein the O and S heteroatoms are protected as esters, carbonates or ethers, and the N-heteroatoms are alkylated or protected as amides or carbamates.

8. Compound according to claim 1 having the formula III

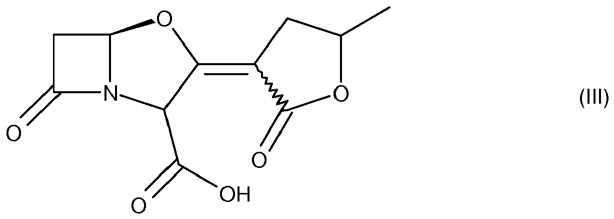

9. Intermediate compound of formula IV

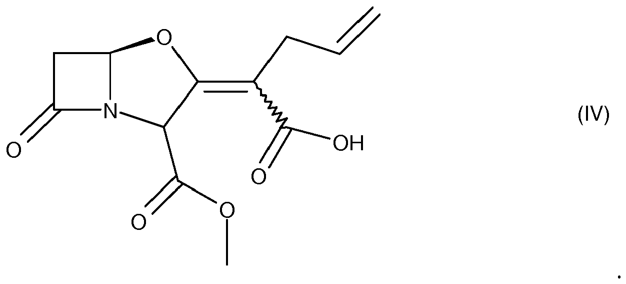

10. Intermediate compound of formula V

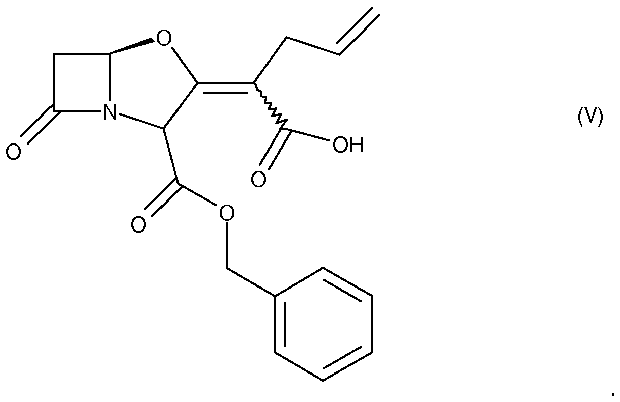

11. Compound according to any one of claims 1 to 8 for use as a medicament.

12. Compound according to any one of claims 9 or 10 for use as a medicament.

13. Pharmaceutical composition comprising a compound of any one of claims 1 to 8, and optionally in addition a β-lactam antibiotic from the class of penicillins or cephalosporins.

14. Pharmaceutical composition comprising a compound of any one of claims 9 or 10, and optionally in addition a β-lactam antibiotic from the class of penicillins or cephalosporins.

## Revendications

1. Composé de formule I 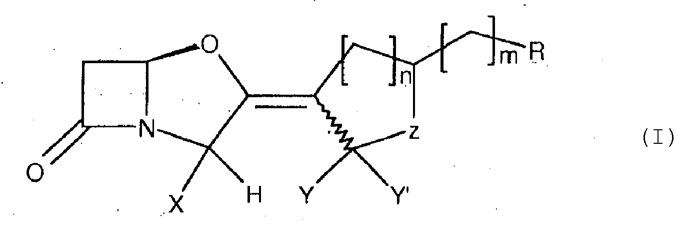 dans laquelle
X représente
un groupe COOH ou un de ses sels,
un groupe COOR₁, où R₁ représente un groupe alkyle en C₁₋₄ éventuellement substitué,
un groupe CONR₂R₃, où R₂ et R₃ sont indépendamment l'un de l'autre un atome H ou un groupe alkyle en C₁₋₅, qui contient éventuellement un ou plusieurs hétéroatomes parmi 0, S et N et/ou est éventuellement substitué, ou
un groupe CON(R₂)-CH(R₄)-R₅, où
R₂ est tel que défini ci-dessus,
R₄ est un groupe qui se trouve habituellement en position α, dans les acides α-aminés et
R₅ est un groupe COOH ou un de ses sels, COOR₁, où R₁ est tel que défini ci-dessus ; ou
un groupe CONR₂R₃, où R₂ et R₃ sont tels que définis ci-dessus,
Y et Y' représentent tous deux un atome H, ou ensemble, un groupe =O,
Z est un atome O, un groupe NR₂ ou N-CH(R₄)-R₅, où R₂, R₄ et R₅ sont tels que définis ci-dessus,
n est égal à 1 ou 2,
m est un nombre de 0 à 4, et
R est un atome H ou
une chaîne saturée ou insaturée avec 1 à 8 atomes de carbone, qui est substituée éventuellement par un cycle non aromatique de 5 ou 6 chaînons qui contient un ou plusieurs hétéroatomes choisis parmi 0, S et N, ou par un groupe aryle, un groupe alkyle ou un groupe cycloalkyle, qui contient éventuellement un ou plusieurs hétéroatomes choisis parmi 0, S et N, et/ou est mono- ou plusieurs fois substitué par des hétéroatomes choisis parmi 0, S et N, par des halogènes Cl, Br, I et F et/ou par un groupe azido N₃.

2. Composé selon la revendication 1, sous la forme d'un sel pharmaceutiquement acceptable, de préférence sous la forme d'un sel de potassium.

3. Composé selon l'une des revendications 1 ou 2, dans lequel R₁ représente un groupe méthyle, de préférence substitué avec un groupe pivaloyloxy.

4. Composé selon l'une des revendications 1 à 3, dans lequel R₂ et R₃ représentent indépendamment l'un de l'autre un groupe alkyle en C₁₋₄ substitué ou un groupe cycloalkyle en C₅₋₆.

5. Composé selon l'une des revendications 1 à 4, dans lequel R₄ représente un groupe méthyle ou un groupe benzyle.

6. Composé selon l'une des revendications 1 à 5, dans lequel R représente une chaîne saturée ou insaturée avec 1 à 8 atomes de carbone, qui est substituée éventuellement par un cycle non aromatique à 5 ou 6 chaînons qui contient un ou plusieurs hétéroatomes choisis parmi 0, S et N, ou par un groupe aryle, un groupe alkyle ou un groupe cycloalkyle.

7. Composé selon l'une des revendications 1 à 6, dans lequel R représente une chaîne saturée ou insaturée avec 1 à 8 atomes de carbone substituée par un ou plusieurs hétéroatomes choisis parmi 0, S et N, où les hétéroatomes 0 et S sont sous forme d'esters, de carbonates et d'éthers, et les hétéroatomes N sont alkylés ou protégés sous forme d'amides ou de carbamates.

8. Composé selon la revendication 1 avec la formule III

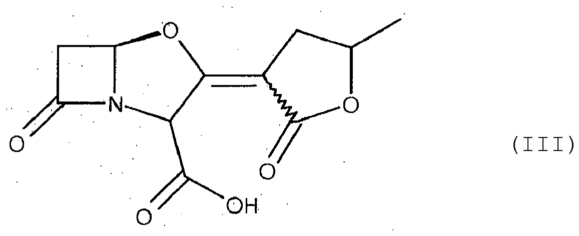

9. Composé intermédiaire de formule IV

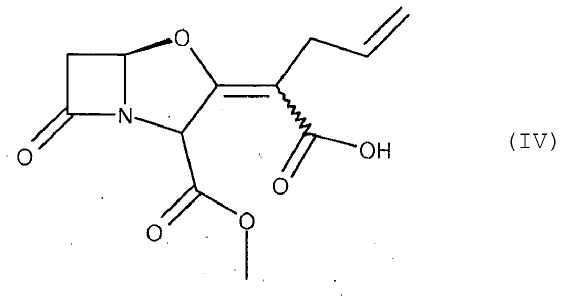

10. Composé intermédiaire de formule V

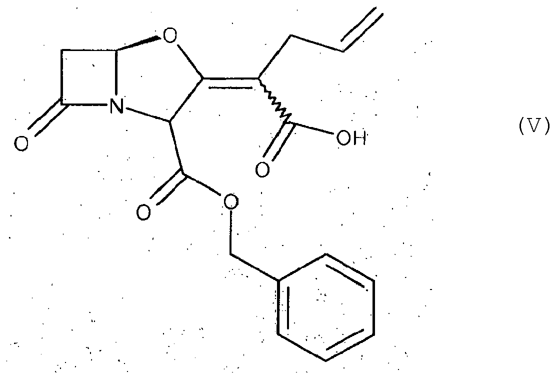

11. Composé selon l'une des revendications 1 à 8 pour une utilisation en tant que médicament.

12. Composé selon l'une des revendications 9 ou 10 pour une utilisation en tant que médicament.

13. Composition pharmaceutique contenant un composé selon l'une des revendications 1 à 8, et de manière complémentaire, éventuellement, un antibiotique de type β-lactame de la classe des pénicillines ou des céphalosporines.

14. Composition pharmaceutique contenant un composé selon l'une des revendications 9 ou 10, et de manière complémentaire, éventuellement, un antibiotique de type β-lactame de la classe des pénicillines ou des céphalosporines.
